# EUROPEAN PATENT APPLICATION

(11) **EP 4 545 083 A1**
(43) Date of publication of application: **30.04.2025**
(21) Application number: 23827232.2
(22) Date of filing: 21.06.2023
(51) Int. Cl.: A61K 33/00, A61K 9/08, A61M 1/28, A61P 43/00

(54) **PERITONEAL DETERIORATION INHIBITING COMPOSITION, PERITONEAL DETERIORATION INHIBITING COMPOSITION KIT, PERITONEAL DIALYSIS FLUID, AND PERITONEAL DIALYSIS FLUID KIT**

(30) Priority: 23.06.2022 JP 2022100790
(71) Applicant: Aichi Medical University, Aichi 480-1195 (JP); Sumitomo Seika Chemicals Co., Ltd., Kako-gun, Hyogo 675-0145 (JP)
(72) Inventor: HATAYAMA Naoyuki, Nagakute-shi, Aichi 480-1195 (JP); NAITO Munekazu, Nagakute-shi, Aichi 480-1195 (JP); FUKUSHIGE Kaori, Nagakute-shi, Aichi 480-1195 (JP); OTSUKA Shun, Nagakute-shi, Aichi 480-1195 (JP); SAKAUE Shigeki, Kako-gun, Hyogo 675-0145 (JP)
(74) Representative: Hoffmann Eitle
(86) International application number: PCT/JP2023/022919
(87) International publication number: WO 2023/249049

(57) **Abstract**

Provided is a composition capable of inhibiting peritoneal deterioration. The peritoneal deterioration inhibiting composition of the present invention includes carbon monoxide.

## Description

### TECHNICAL FIELD

The present invention relates to a peritoneal deterioration inhibiting composition, a peritoneal deterioration inhibiting composition kit, a peritoneal dialysate, and a peritoneal dialysate kit.

### BACKGROUND ART

Artificial dialysis has been performed as a treatment method for supplementing the renal function of patients with decreased renal function. Artificial dialysis includes hemodialysis and peritoneal dialysis. Hemodialysis takes about four to five hours per session, and about three sessions are needed per week. In contrast, peritoneal dialysis can be realized by replacing a dialysate once a day while sleeping, and has an advantage of improving QOL of patients (Non-Patent Literature 1).

### Citation List

### Non-Patent Literature

Non-Patent Literature 1: Qin Zhou et al., "Preventing peritoneal membrane fibrosis in peritoneal dialysis patients," Kidney International, Volume 90, Issue 3, 2016, Pages 515-524

### SUMMARY OF INVENTION

### Technical Problem

However, there is the problem that patients treated with peritoneal dialysis suffer from peritoneal dysfunction such as a decrease in the water removal function over time, bringing about an unfeasible condition for peritoneal dialysis six to seven years after the start of peritoneal dialysis. In such cases, the patient discontinues peritoneal dialysis and moves on to hemodialysis. Accordingly, in order to extend the period of peritoneal dialysis, there is a need for a composition capable of inhibiting peritoneal deterioration.

Hence, it is an object of the present invention to provide a composition capable of inhibiting peritoneal deterioration.

### Solution to Problem

To achieve the above object, the present invention provides a peritoneal deterioration inhibiting composition (hereinafter also referred to as the "composition"), including carbon monoxide.

The present invention also provides a peritoneal deterioration inhibiting composition kit (hereinafter also referred to as the "composition kit"), including:
a peritoneal deterioration inhibiting composition, and
an other component, wherein
the peritoneal deterioration inhibiting composition and the other component are placed separately from each other, and
the peritoneal deterioration inhibiting composition is the composition of the present invention.

The present invention also provides a peritoneal dialysate, including the composition of the present invention.

The present invention also provides a peritoneal dialysate kit, including:
a composition, and
a peritoneal dialysate, wherein
the composition and the peritoneal dialysate are placed separately from each other, and the composition is the composition of the present invention.

The present invention also provides a pharmaceutical composition for use in prevention or inhibition of a disease resulting from peritoneal dialysis (hereinafter also referred to as the "pharmaceutical composition"), including carbon monoxide.

The present invention also provides a pharmaceutical composition kit for use in prevention or inhibition of a disease resulting from peritoneal dialysis (hereinafter also referred to as the "pharmaceutical kit" or the "pharmaceutical composition kit"), including:
a composition, and
an other component, wherein
the composition and the other component are placed separately from each other, and
the composition is the composition of the present invention.

### Advantageous Effects of Invention

The composition of the present invention can inhibit peritoneal deterioration.

### BRIEF DESCRIPTION OF DRAWINGS

[FIG. 1] FIG. 1 is a cross-sectional view showing an example of a double-chamber container accommodating the composition of the present invention and an other component.
[FIG. 2] FIG. 2 is a schematic view showing a piece of equipment for producing micro bubbles in Example 1.
[FIG. 3] FIG. 3 is a graph showing the drainage amount in Example 1.
[FIG. 4] FIG. 4 shows photographs of peritoneal thickening stained using tissue staining in Example 1.
[FIG. 5] FIG. 5 shows photographs of inflammatory cells stained using tissue staining in Example 1.
[FIG. 6] FIG. 6 shows photographs of the results of CD68 (macrophage marker) staining by immunohistochemical staining in Example 1.
[FIG. 7] FIG. 7 is a graph showing macrophage positive areas (%) in Example 1.
[FIG. 8] FIG. 8 shows photographs of the results of CD31 (vascular endothelial cell marker) staining by immunohistochemical staining in Example 1.
[FIG. 9] FIG. 9 is a graph showing vascular endothelial cell-positive areas (%) in Example 1.
[FIG. 10] FIG. 10 shows photographs of the results of Lyve-1 (lymphatic vessel marker) staining by immunohistochemical staining in Example 1.
[FIG. 11] FIG. 11 is a graph showing the results of the lymphatic vessel-positive areas (%) in Example 1.
[FIG. 12] FIG. 12 shows graphs of the expression level of each gene in Example 1.
[FIG. 13] FIG. 13 is a graph showing the peritoneal fluid amount in Example 2.
[FIG. 14] FIG. 14 is a graph showing the thickness of the peritoneal surface (peritoneal thickness) in Example 2.
[FIG. 15] FIG. 15 is a graph showing the expression levels of IL-6 genes in Example 3.
[FIGs. 16A to 16H] FIGs. 16A to 16H are graphs showing the peritoneal fluid amount in Example 4. FIG. 16A shows the result of CO-UFB. FIG. 16B shows the result of CO-UFB 1/4. FIG. 16C shows the result of CO-UFB 1/10. FIG. 16D shows the result of CO-UFB 1/50. FIG. 16E shows the result of CO-dis. FIG. 16F shows the result of CO-dis 1/4. FIG. 16G shows the result of CO-dis 1/10. FIG. 16H shows the result of CO-dis 1/50.

### DESCRIPTION OF EMBODIMENTS

### <Definition>

As used herein, "peritoneum" refers to a membrane covering some or all of the organs of the abdomen. The peritoneum is composed of the parietal peritoneum and the visceral peritoneum (including the diaphragm). Under normal conditions, the peritoneum covers the outer surface, and is composed of the mesothelium, itself made up of mesothelial cells, and the submesothelial layer composed of connective tissues.

As used herein, "peritoneal deterioration" refers to a decrease in peritoneal function and/or change in peritoneal form. The peritoneal deterioration is preferrably the one occurring in peritoneal dialysis. "Decrease in the peritoneal function" refers to a decrease or failure in the ultrafiltration function of the peritoneum and/or acceleration in the peritoneal permeability. A "change in peritoneal form" refers to fibrous thickening of the peritoneum and/or sclerous thickening of the peritoneum. "Peritoneal deterioration" can be evaluated by examining peritoneal function and/or peritoneal form of the subject. Peritoneal function can be evaluated, for example, through the use of a Peritoneal Equilibration Test (PET). Peritoneal function may be evaluated based on the water removal function in accordance with Example 1, described later. Peritoneal form can be evaluated, for example, by biopsy of the peritoneum of the subject, or the like.

As used herein, "peritoneal fibrosis" refers to a state where the extracellular matrix such as collagen is deposited in the peritoneum.

As used herein, "inhibition of peritoneal deterioration" may refer to prevention, suppression, or cessation of decrease in peritoneal function, inhibition or cessation of progression of decrease in peritoneal function, and/or improvement or remission (relief) of the peritoneal function, or may refer to prevention, inhibition, suppression, or cessation of change in peritoneal form, inhibition or cessation of progression of change in peritoneal form, and/or improvement or normalization of peritoneal form that has changed.

As used herein, "inhibition of peritoneal fibrosis" refers to significantly inhibiting peritoneal fibrosis in the subject. "Inhibition of peritoneal fibrosis" may also be referred to as, for example, prevention, suppression or cessation of peritoneal fibrosis, inhibition or cessation of progression of peritoneal fibrosis, and/or improvement or normalization of the fibrosed peritoneum, in the subject. "Inhibition of peritoneal fibrosis" can be evaluated by, for example, examining whether fibrosis is significantly inhibited under the conditions that induce peritoneal fibrosis. Specifically, for example, in the evaluation of "inhibition of peritoneal fibrosis" under the conditions that induce peritoneal fibrosis, when the fibrosis in a treated group treated with a test substance is significantly inhibited compared to a control group untreated with the test substance, or a control group treated with a substance (control substance) having no inhibitory activity for peritoneal fibrosis, the test substance can be evaluated as having peritoneal fibrosis-inhibitory activity. Inhibition of peritoneal fibrosis may be evaluated on the basis of peritoneal thickening in a model, with peritoneal deterioration induced by a chlorhexidine gluconate solution, in accordance with Example 1 (4) described later.

As used herein, "inhibition of peritoneal inflammation" refers to significantly inhibiting peritoneal inflammation of the subject. "Inhibition of peritoneal inflammation" may also be referred to as, for example, prevention, suppression, or cessation of peritoneal inflammation, and/or inhibition or cessation of progression (exacerbation) of peritoneal inflammation, in the subject. "Inhibition of peritoneal inflammation" can be evaluated, for example, by examining whether inflammation is significantly inhibited under the conditions that induce peritoneal inflammation. Specifically, for example, in the evaluation of "inhibition of peritoneal inflammation" under the conditions that induce peritoneal inflammation, when inflammation in a treated group treated with a test substance is significantly inhibited compared to a control group untreated with the test substance, or a control group treated with a substance (control substance) having no inhibitory activity for the peritoneal inflammation, the test substance can be evaluated as having peritoneal inflammation-inhibitory activity. The inhibition of peritoneal inflammation may be evaluated based on the infiltration of macrophages into the peritoneum in a model with peritoneal deterioration induced by a chlorhexidine gluconate solution in accordance with Example 1 (5) described later, and/or based on the expression level of peritoneal inflammatory cytokines in the peritoneum described in Example 1 (6).

As used herein, "inhibition of angiogenesis" refers to significantly inhibiting angiogenesis in the peritoneum. Specifically, for example, in the evaluation of "inhibition of angiogenesis" under the conditions that induce angiogenesis in the peritoneum, when angiogenesis in a treated group treated with a test substance is significantly inhibited compared to a control group untreated with a test substance, or a control group treated with a substance (control substance) having no inhibitory activity for the angiogenesis in the peritoneum, the test substance can be evaluated as having angiogenesis inhibitory activity. The inhibition of angiogenesis in the peritoneum may be evaluated based on a positive area of a vascular endothelial cell marker in the peritoneum of a model with peritoneal deterioration induced by a chlorhexidine gluconate solution, in accordance with Example 1 (5) described later.

As used herein, "inhibition of lymphangiogenesis" refers to significantly inhibiting lymphangiogenesis in the peritoneum. Specifically, for example, in the evaluation of "inhibition of lymphangiogenesis" under the conditions that induce lymphangiogenesis in the peritoneum, when lymphangiogenesis in a treated group treated with a test substance is significantly inhibited compared to a control group untreated with a test substance, or a control group treated with a substance (control substance) having no inhibitory activity for the lymphangiogenesis in the peritoneum, the test substance can be evaluated as having lymphangiogenesis inhibitory activity. The inhibition of the lymphangiogenesis in the peritoneum may be evaluated based on a positive area of a lymphatic endothelial cell marker in the peritoneum of a model with peritoneal deterioration induced by a chlorhexidine gluconate solution, in accordance with Example 1 (5) described later. In the evaluation of lymphangiogenesis, the peritoneum is preferably the diaphragm.

As used herein, "positive (+)" refers to the result of the detection of a higher signal by an analysis method such as immunohistochemical staining using an antigen-antibody reaction, than a negative control cell that does not express the antigen, or a negative control reaction using the antibody that does not react with the antigen.

As used herein, "negative (-)" refers to the result of the detection of signal equal to or lower than a negative control cell that does not express the antigen, or a negative control reaction using the antibody that does not react with the antigen.

As used herein, a "micro bubble" refers to a closed microspace composed of gas, which is surrounded by other than gas. The "micro bubble" may be referred to as, for example, a minute bubble. The micro bubble may be, for example, a fine bubble. The fine bubble generally refers to a micro bubble having a bubble diameter of less than 100 µm. The bubble diameter referes to a sphere equivalent diameter of the bubble. The bubble diameter may be an average diameter (arithmetic average diameter) of a micro bubble obtained by a measurement method described later. The fine bubble (FB) may be a microbubble or an ultrafine bubble (UFB). The microbubble generally refers to a micro bubble having a bubble diameter of 1 µm or more and less than 100 µm. The ultrafine bubble generally refers to a micro bubble having a bubble diameter of less than 1 µm. A bubble diameter of the ultrafine bubbles is, for example, 1 nm or more, less than 1000 nm, 1 to 750 nm, or 1 to 500 nm.

As used herein, a "subject" refers to an animal, or a cell, tissue or organ derived from an animal, and is used in a sense particularly including a human. "Animal" refers to a human and a non-human animal. Examples of a non-human animal include mammals such as mice, rats, rabbits, dogs, cats, cows, horses, pigs, monkeys, dolphins, and red deer.

As used herein, "treatment" refers to therapeutic treatment and/or prophylactic treatment. As used herein, "curing" refers to curing, healing, prevention, suppression, remission, or improvement of a disease, a disease state, or a failure, or to the cessation, suppression, reduction, or delay of the progress of a disease, a disease state, or a failure. As used herein, "prevention" refers to a decrease in the possibility of developing a disease or a disease state, or to a delay in the development of a disease or a disease state. The "curing" may entail, for example, curing a patient who develops a disease of interest, or curing a model animal of the disease of interest.

Hereinafter, the present invention will be described with reference to examples, but the present invention is not limited to the following examples, and can be implemented with arbitrary modification. The descriptions in the present invention can be incorporated with each other unless otherwise specified. In the present specification, the use of "to" is intended to include numerical values or physical values before and after the expression. In the present specification, the use of "A and/or B" is intended to include "only A", "only B", and "both A and B".

### <Peritoneal deterioration inhibiting composition>

The present invention provides a composition that inhibits peritoneal deterioration. The peritoneal deterioration inhibiting composition of the present invention includes carbon monoxide. The composition of the present invention is characterized by the inclusion of carbon monoxide (CO), and other configurations and conditions are not particularly limited. The composition of the present invention can inhibit peritoneal deterioration. The composition of the present invention includes CO, and thus can be directly administered into a body of an administration subject, for example, peritoneally, intraperitoneally, or the like.

The carbon monoxide is, for example, present in a medium. The carbon monoxide may be present in the medium in a state of being separated from the medium, or in a state of being merged with the medium. When the carbon monoxide is present in a state of being separated from the medium, the carbon monoxide is present in a state of being able to be divided or distinguished from the medium, for example. In this case, for example, the carbon monoxide is present in spaces surrounded by the medium, and specifically, for example, is present as bubbles. The bubbles may be, for example, micro bubbles. When the carbon monoxide is present in a state of being merged with the medium, the carbon monoxide is present, for example, in a state of being unable to be divided or distinguished from the medium. In this case, the carbon monoxide is present with being dissolved in the medium. When the medium is a liquid solvent, the medium in which the carbon monoxide is dissolved may be referred to as, for example, a carbon monoxide-dissolved liquid.

The medium may be, for example, a liquid or a solid. The liquid may be, for example, a solvent such as an aqueous solvent including water, an oily solvent, or a mixed solvent of these. Further, the liquid includes sol. The solid may be, for example, a solid obtained by solidifying the liquid. Further, the solid includes gel. Examples of a liquid include a physiological saline solution; a buffer solution such as a phosphate buffer solution; an infusion solution such as an extracellular fluid and an intracellular fluid; water such as distilled water and pure water; a cell culture solution such as DMEM and RPMI1640; and an organ preservative solution. The solid may be, for example, a solidified product of the liquid.

The micro bubbles are dispersed in the medium. Thus, it can be said that a gas component of the micro bubble is surrounded by the medium. The gas component of the micro bubble and the medium are preferred to be directly in contact with each other. The micro bubbles are dispersed in the entire or a part of the medium. In the latter case, it can also be said that the micro bubbles are localized in a part of the medium.

The composition of the present invention includes carbon monoxide as a gas component. The composition of the present invention may include only carbon monoxide (CO) as gas (gas component), or may further include other gas. The CO may also be referred to as an active component in the composition of the present invention, for example. Examples of an other gas include biogases such as nitric oxide (NO), hydrogen sulfide (H₂S), and hydrogen (H₂); rare gases such as helium (He), argon (Ar), krypton (Kr), and xenon (Xe); carbon dioxide (CO₂), nitrous oxide (N₂O), carbon dioxide (CO₂), nitrogen (N₂), methane (CH₄), ethane (CH₃CH₃), propane (CH₃CH₂CH₃), fluoromethane (CH₃F), difluoromethane (CH₂F₂), carbon tetrafluoride (CF₄), ethylene oxide (C₂H₄O), and air. As used herein, "biogas" refers to gas including carbon monoxide (CO), nitric oxide (NO), hydrogen sulfide (H₂S), or hydrogen (H₂), or mixed gas including two or more of these gases. When the composition of the present invention includes two or more types of gas components, the gas component other than CO is preferrably a gas component that does not react with CO, such as the rare gases and nitrogen for example. Regarding the carbon monoxide, a case where the gas is only air is to be excluded. As used herein, "air" refers to, for example, air (atmosphere) used in producing the composition of the present invention. In the composition of the present invention, when carbon monoxide and the other gas are gases with medical gas grade, the carbon monoxide and the other gas are preferrably gas derived from medical gas. The other gas component may be present in the medium, for example, in a state that is the same as or different from CO. Specifically, for example, when the carbon monoxide forms bubbles in the medium, the other gas component may form bubbles together with or separately from the carbon monoxide in the medium, or may be dissolved in the medium. When the carbon monoxide is dissolved in the medium, the other gas component may form bubbles in the medium, or may be dissolved in the medium together with the carbon monoxide.

In the composition of the present invention, the content of the carbon monoxide can be set depending on, for example, a dosage to an administration subject described later. The lower limit of the content (concentration) of the carbon monoxide may be, for example, 0.01 µmol/L or more, 0.1 µmol/L or more, 1 µmol/L or more, 10 µmol/L or more, 15 µmol/L, 50 µmol/L or more, 75 µmol/L or more, or 100 µmol/L or more. The upper limit of the content (concentration) of the carbon monoxide may be, for example, 5 mmol/L or less, 1 mmol/L or less, 0.75 mmol/L or less, 0.5 mmol/L or less, or 0.25 mmol/L or less. The content (concentration) of the carbon monoxide in the composition of the present invention is within the range of, for example, 0.01 µmol/L to 5 mmol/L, 0.1 µmol/L to 1 mmol/L, 1 µmol/L to 1 mmol/L, 10 µmol/L to 1 mmol/L, or 100 µmol/L to 1 mmol/L.

When the composition of the present invention includes carbon monoxide as bubbles and/or micro bubbles, the density of the bubbles and/or the micro bubbles refers to the number of bubbles and/or micro bubbles to the volume of the medium. "Density" may also be referred to as a number concentration. The lower limit of the density of the bubbles and/or the micro bubbles is, for example, 5×10⁵ bubbles/mL, 1×10⁶ bubbles/mL, 5×10⁶ bubbles/mL, 1×10⁷ bubbles/mL, 5×10⁷ bubbles/mL, 1×10⁸ bubbles/mL, 5×10⁸ bubbles/mL, or 1×10⁹ bubbles/mL, and preferably is 1×10⁶ bubbles/mL, 5×10⁶ bubbles/mL, 1×10⁷ bubbles/mL, 5×10⁷ bubbles/mL, 1×10⁸ bubbles/mL, or 5×10⁸ bubbles/mL. The upper limit of the density of the bubbles and/or the micro bubbles is, for example, 1.5×10⁹ bubbles/mL, 2×10⁹ bubbles/mL, 3×10⁹ bubbles/mL, 5×10⁹ bubbles/mL, 7×10⁹ bubbles/mL, 9×10⁹ bubbles/mL, 1×10¹⁰ bubbles/mL, 5×10¹⁰ bubbles/mL, 1×10¹¹ bubbles/mL, 5×10¹¹ bubbles/mL, 1×10¹² bubbles/mL, or 5×10¹² bubbles/mL. The density of the bubbles and/or the micro bubbles is within the range of, for example, 5×10⁵ bubbles/mL to 5×10¹² bubbles/mL, 5×10⁵ bubbles/mL to 1×10¹² bubbles/mL, 5×10⁵ bubbles/mL to 5×10¹¹ bubbles/mL, 5×10⁵ bubbles/mL to 1×10¹¹ bubbles/mL, 5×10⁵ bubbles/mL to 5×10¹⁰ bubbles/mL, 5×10⁵ bubbles/mL to 1×10¹⁰ bubbles/mL, 1×10⁶ bubbles/mL to 9×10⁹ bubbles/mL, 5×10⁶ bubbles/mL to 9×10⁹ bubbles/mL, 1×10⁷ bubbles/mL to 7×10⁹ bubbles/mL, 5×10⁷ bubbles/mL to 7×10⁹ bubbles/mL, 1×10⁸ bubbles/mL to 5×10⁹ bubbles/mL, 5×10⁸ bubbles/mL to 5×10⁹ bubbles/mL, 1×10⁹ bubbles/mL to 3×10⁹ bubbles/mL, 5×10⁸ bubbles/mL to 2×10⁹ bubbles/mL, or 5×10⁸ bubbles/mL to 1. 5 × 10⁹ bubbles/mL.

The density, the bubble diameter, and the average diameter (hereinafter also referred to as "properties") of the bubbles and/or the micro bubbles can be appropriately measured depending on the medium in which the bubbles and/or micro bubbles are dispersed. When the bubbles and/or micro bubbles are dispersed in a liquid medium, the properties of the bubbles and/or micro bubbles can be calculated by analyzing the bubbles in the composition of the present invention by a particle tracking analysis method. The particle tracking analysis method can be performed, for example, in accordance with Example 1 described later, using NANOSIGHT^{™} NS300 (manufactured by Malvern Instruments). The properties of the bubbles and/or the micro bubbles may be calculated by an analysis method other than the particle tracking analysis method. In this case, the properties of the bubbles and/or the micro bubbles obtained by the other analysis method satisfy the above exemplifications when converted into the calculated values obtained by the particle tracking analysis method. When the bubbles and/or the micro bubbles are dispersed in a solid medium, the properties of the bubbles and/or micro bubbles can be calculated based on the properties of bubbles and/or micro bubbles in a liquid before solidification of the medium, and the properties of the bubbles and/or the micro bubbles in a liquid obtained by dissolving the solid medium.

The proportion of CO in the gas is, for example, more than 0%, 100% or less, 10 to 100%, 20 to 100%, 30 to 100%, 40 to 100%, 50 to 100%, 60 to 100%, 70 to 100%, 80 to 100%, 90 to 100%, 95 to 100%, 96 to 100%, 97 to 100%, 98 to 100%, 99 to 100%, and preferably 90 to 100%.

The composition of the present invention preferably exhibits any one, two, three or four activities of the following (1) to (4). The activities of the following (1) to (4) can be evaluated using the method described above.
(1) Peritoneal fibrosis inhibitory activity
(2) Peritoneal inflammation inhibitory activity
(3) Peritoneal angiogenesis inhibitory activity
(4) Peritoneal lymphangiogenesis inhibitory activity

Regarding (1), for example, based on the peritoneal thickness of the control group, the composition of the present invention has an activity of inhibiting the peritoneal thickeness by 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more, in an assay using the peritoneal deterioration model, 9 or 16 days after induction of the peritoneal deterioration.

Regarding (2), for example, based on the expression level of the inflammatory cytokine in the peritoneum of the control group, the composition of the present invention has an activity of inhibiting the expression level of inflammatory cytokine in a group administered the composition of the present invention by 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more, in an assay using the peritoneal deterioration model, 9 or 16 days after induction of the peritoneal deterioration.

Regarding (3), for example, based on the are of the positive area of the vascular endothelial cell marker in the peritoneum of the control group, the composition of the present invention has an activity of inhibiting the area of the positive area of a vascular endothelial cell marker by 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more, in an assay using the peritoneal deterioration model, 9 or 16 days after induction of the peritoneal deterioration.

Regarding (4), for example, based on the area of the positive area of the lymphatic endothelial cell marker in the peritoneum of the control group, the composition of the present invention has an activity of inhibiting the area of the positive area of a lymphatic endothelial cell marker by 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more, in an assay using the peritoneal deterioration model, 9 or 16 days after induction of the peritoneal deterioration.

The composition of the present invention can be produced by a method for producing a medium with dissolved gas, or a method for producing a medium including bubbles, depending on the state of CO in the composition. When the CO is dissolved in the medium in the composition of the present invention, the composition of the present invention can be produced, for example, by mixing the medium and the gas after encapsulating a medium such as a physiological saline solution and a gas in a sealable container. The volume ratio between the medium and the gas can be set depending on, for example, the content of the gas in the medium, and the volume ratio (S:G) between the medium (S) and the gas (G) is, for example, 1:0.01 to 100, 1:0.1 to 10, or 1:0.5 to 5. The mixing time may be, for example, 1 minute to 24 hours, 10 minutes to 12 hours, or 20 minutes to 1 hour. When the CO forms bubbles in the composition of the present invention, the composition of the present invention can be produced, for example, by a method for producing micro bubbles such as fine bubbles using arbitrary gas. Therefore, the method for producing the composition of the present invention includes a process of producing bubbles for producing micro bubbles using CO-containing gas and a medium, for example. Specifically for example, when the composition of the present invention is liquid, the liquid composition can be produced, for example, using the CO-containing gas, the medium, and a micro bubble production equipment which is swirling flow type, ejector type, venturi type, static mixer type, microporous type, pressurizing dissolving type, or ultrasonic cavitation type. When the composition of the present invention is a solid, the solid composition can be produced by solidifying the liquid composition by a known method. When the solid is a gel, the gel composition can be produced, for example, by mixing the liquid composition with a gelling agent. At the beginning of the process of producing bubbles, the CO-containing gas may be gas, liquid, or solid. The CO-containing gas may include two or more types of gas. In this case, each of the gases may be separately subjected to the process of producing bubbles, or all of or a part of the CO-containing gases may be simultaneously subjected to the process of producing bubbles. Specifically, for example, when the gas includes CO and gas other than CO, the CO and the gas other than CO may be introduced simultaneously or separately.

The composition of the present invention may be used, for example, *in vivo* or *in vitro.* The composition of the present invention can be used, for example, as a research reagent or as a medicine. In the latter case, the composition of the present invention may also be referred to as a pharmaceutical or a pharmaceutical composition.

An administration subject (subject) of the composition of the present invention is not particularly limited. When using the composition of the present invention *in vivo,* the administration subject can, for example, incorporate the above description of the subject. When using the composition of the present invention *in vitro,* the administration subject may be, for example, cells, tissue, organs, or the like. The cells may be, for example, cells collected from a living body, cultured cells, or the like. The tissue or organs may be, for example, tissue (living tissue) or organs collected from a living body. Examples of cells include peritoneal endothelial cells and peritoneal mesothelial cells.

The administration subject for the composition of the present invention is preferrably a subject scheduled to undergo peritoneal dialysis, a subject who is undergoing peritoneal dialysis, a subject who has undergone peritoneal dialysis, and/or a subject suspected of deterioration of peritoneal function due to peritoneal dialysis.

The use conditions (administration conditions) of the composition of the present invention are not particularly limited, and for example, the administration form, the administration period, the dosage, and the like can be appropriately set depending on the type of the administration subject, or the like.

The dosage of the composition of the present invention is not particularly limited, and is, for example, a therapeutically effective amount. When using the composition of the present invention *in vivo,* the dosage of the composition can be appropriately determined, for example, depending on a type, a symptom, age, an administration method, and the like of the administration subject. Specifically, for example, when intraperitoneally or intravenously administering a human or a mouse with a composition having the micro bubbles at the density of 1×10⁸ bubbles/mL (carbon monoxide content: about 0.0002 mm³ /mL) to 5×10¹² bubbles/mL (carbon monoxide content: about 10 mm³ /mL), or with a composition having the carbon monoxide at a content (concentration) of 0.01 µmol/L to 5 mmol/L, the total dosage of the composition per day is, for example, 1 to 80 mL/kg body weight, or 20 to 80 mL/kg body weight. In this case, the number of doses of the composition of the present invention per day is, for example, 1 to 5 doses or 1 to 3 doses, and preferably, 1 dose. When intraperitoneally administering a human a composition having the density of the micro bubbles of 1×10⁸ bubbles/mL to 5×10¹² bubbles/mL, or a composition having the content (concentration) of the carbon monoxide of 0.01 µmol/L to 5 mmol/L, as a peritoneal dialysate, the dosage of the composition per dose is 2 to 5 L, and the total dosage per day is, for example, 2 to 30 L, 5 to 20 L, or 8 to 18 L. In this case, the number of doses of the composition of the present invention per day is, for example, 1 to 15 doses, 1 to 13 doses, or 3 to 10 doses. When administering a human or a mouse a composition having the micro bubbles at the density of 1×10⁸ bubbles/mL to 5×10¹² bubbles/mL, or a composition having the carbon monoxide at the content (concentration) of 0.01 µmol/L to 5 mmol/L, for prophylactic use, the dosage of the composition per day is, for example, 0.00001 to 500 mL per dose. In this case, the number of doses of the composition of the present invention per day is, for example, 1 to 5 doses, or 1 to 3 doses, and preferably, 1 dose. The content of the gas component in the composition is not particularly limited, and can be appropriately set depending on, for example, the dosage per day described above. The composition of the present invention may be administered continuously or discontinuously, for example. The discontinuous administration can also be referred to as "intermittent administration," for example. The composition of the present invention may be administered, for example, at predetermined intervals. The predetermined intervals may be substantially equal intervals or equal intervals, or may be unequal intervals. The predetermined interval may be, for example, an interval of 8 to 12 hours, an interval of one day, or the like.

The administration form of the composition of the present invention is not particularly limited. When administering the composition of the present invention *in vivo,* the composition may be administered orally or parenterally. Examples of parenteral administration include intravenous injection (intravenous administration), intramuscular injection (intramuscular administration), transdermal administration, subcutaneous administration, intradermal administration, enteral administration, rectal administration, vaginal administration, nasal administration, pulmonary administration, intraperitoneal administration, and topical administration. When intraperitoneally administering the composition of the present invention, the composition administered intraperitoneally may be collected from the peritoneal cavity.

The formulation type of the composition of the present invention is not particularly limited, and can be appropriately determined depending on, for example, the administration form. The formulation type may be, for example, a liquid form or a solid form. Specific examples of formulation type include a formulation for oral administration such as a modified release formulation (an enteric formulation, a sustained release formulation, etc.), a capsule, an oral liquid agent (an elixir, a suspension, an emulsion, a fragrance, a limonade, etc.), a syrup (a syrup agent, etc.), granules (foaming granules, fine granules, etc.), powders, a tablet (an orally disintegrable tablet, a chewable tablet, a foamable tablet, a dispersible tablet, a soluble tablet, a coated tablet, etc.), a pill, and an oral jelly; a formulation for an oral cavity application form such as a tablet for applying to the oral cavity (a gum, a sublingual formulation, a lozenge, a drop, a buccal tablet, an adhesive tablet, etc.), an oral spray, an oral semisolid formulation, a gargling agent; a formulation for injection such as an injection agent (an agent for implementing injection, a sustainable injection agent, a infusion agent (a formulation for drip infusion, etc.), a lyophilized injection agent, powder for injections, a filled syringe agent, a cartridge agent, etc); a formulation for dialysis such as a dialysis agent (a peritoneal dialysis agent, a hemodialysis agent); a formulation for bronchial and pulmonary applications such as inhalants (inhalation aerosols, an inhalation solution, inhalation powders, etc.); a formulation for eye administration such as an eye ointment and an eye lotion; a formulation for ear administration such as an ear drop agent; a formulation for nasal application such as a nasal drop agent (a nasal drop liquid, nasal drop powders, etc.); a formulation for rectal application such as a suppository, a rectal semisolid formulation, and an enema agent; a formulation for vaginal application such as a vaginal suppository and a vaginal tablet, and a formulation for skin application such as a liquid for external use (an alcohol agent, a liniment, a lotion, etc.), cream, gel, a solid formulation for external use (powders for external use, etc.), spray (aerosols for external use, pump spray, etc.), a patch (a tape, a cataplasm, etc.), and an ointment. When orally administering the composition of the present invention, the formulation type may be, for example, a tablet, a coated tablet, a pill, fine granules, granules, powders, a capsule, a liquid, a syrup, an emulsion, a suspension, and the like. When parenterally administering the composition of the present invention, the formulation type may be, for example, a formulation for injection, a formulation for drip infusion, and the like. When transdermally administering the composition of the present invention, the formulation type may be, for example, a medicine for external use such as a patch, a coating agent, an ointment, cream, and a lotion.

The composition of the present invention may include an additive as needed, for example. When using the composition of the present invention as a pharmaceutical or pharmaceutical composition, the additive is preferred to include a pharmaceutically acceptable additive or a pharmaceutically acceptable carrier. The additive is not particularly limited, and examples thereof include an osmotic pressure adjusting agent such as a salt, a base material, an excipient, a colorant, a lubricant, a binder, a disintegrator, a stabilizer, a coating agent, a preservative, a pH adjusting agent, and a taste-and-smell-correcting agent such as a flavoring agent. In the present invention, the blending amount of the additive is not particularly limited as long as it does not impair the function of CO.

Examples of an excipient include a sugar derivative such as lactose, lactose hydrate, sucrose, glucose, mannitol, and sorbitol; a starch derivative such as corn starch, potato starch, alpha starch, and dextrin; a cellulose derivative such as crystalline cellulose; gum arabic; dextran; an organic excipient such as pullulan, and an inorganic excipient such as a silicate derivative, including a light silicic acid anhydride, a synthetic aluminum silicate, a calcium silicate, and a metasilicate; a phosphate such as a calcium hydrogenphosphate; a carbonate such as a calcium carbonate, and a sulfate such as a calcium sulfate. The colorant may be, for example, yellow iron sesquioxide and the like. Examples of a lubricant include a metal stearate such as stearic acid, a calcium stearate, and a magnesium stearate; talc; polyethylene glycol; silica, and hydrogenated vegetable oil. Examples of a taste-and-smell-correcting agent include a flavoring agent such as cocoa powder, menthol, aromatic powder, peppermint oil, borneol, and cinnamon powders, a sweetener, and an acidulant. Examples of a binder include hydroxypropylcellulose, hydroxypropylmethylcellulose, polyvinylpyrrolidone, and macrogol. Examples of a disintegrant include a cellulose derivative such as carboxymethyl cellulose and carboxymethyl cellulose calcium; chemically modified starch such as carboxymethyl starch, sodium carboxymethyl starch, cross-linked polyvinyl pyrrolidone, and sodium starch glycolate, and chemically modified celluloses. Examples of a stabilizer include paraoxybenzoic acid esters such as methyl paraben and propyl paraben; alcohols such as chlorobutanol, benzyl alcohol and phenylethyl alcohol; benzalkonium chloride; phenols such as phenol and cresol; thimerosal; dehydroacetic acid, and sorbic acid. Examples of a coating agent include macrogol such as hypromellose and macrogol 6000, talc, and titanium oxide.

The composition of the present invention can inhibit, for example, peritoneal deterioration of an administration subject. Therefore, the composition of the present invention can be suitably used, for example, as a composition for inhibiting peritoneal deterioration caused by peritoneal dialysis, preventing or inhibiting peritoneal inflammation and/or peritoneal fibrosis resulting from peritoneal deterioration, and preventing or inhibiting encapsulating peritoneal sclerosis resulting from the peritoneal deterioration.

### < Peritoneal deterioration inhibiting composition kit>

In one aspect, the present invention provides a composition kit that inhibits peritoneal deterioration. As described above, a peritoneal deterioration inhibiting composition kit of the present invention includes the peritoneal deterioration inhibiting composition of the present invention and an other component, wherein the peritoneal deterioration inhibiting composition and the other component are placed separately from each other, and the peritoneal deterioration inhibiting composition is the peritoneal deterioration inhibiting composition of the present invention. The composition kit of the present invention is characterized by including the composition of the present invention, and other configurations and conditions are not particularly limited. The composition kit of the present invention can control the dosage of the composition, and thus can control the dosage of CO. The composition kit of the present invention can incorporate the description of the composition of the present invention.

The other component is not particularly limited, and can be appropriately determined depending on what is included in the composition and the purpose of administration to an administration subject, and examples thereof include an additive, a medicine, and a nutritional agent. Examples of a medicine include antibiotics. When the osmotic pressure of the composition is not adjusted, the other component is preferred to include an osmotic pressure adjusting agent (substance). Examples of a osmotic pressure adjusting substance include a sugar such as glucose and icodextrin; a salt (electrolyte) such as sodium chloride, sodium lactate, calcium chloride, calcium chloride, sodium bicarbonate, and magnesium chloride; an amino acid, and a protein. Examples of a nutritional agent include a sugar such as glucose, and vitamins. The other component may be a solid or a liquid. In the former case, the other component is preferred to be placed in an undissolved state in a solvent or the like, and configured to be dissolved when mixed with the composition, for example. In the latter case, the other component is preferred to be dissolved in a solvent, for example.

In the composition kit of the present invention, the composition and the other component are placed separately from each other. That is, the composition and the other component are placed in an unmixed state or in a non-contact state. Specifically, the composition and the other component are placed at different positions in the container used for accommodating these.

In the composition kit of the present invention, the composition and the other component are preferred to be accommodated in a container. In this case, the container includes a plurality of chambers, and the composition and the other component are accommodated in different chambers. That is, the container is preferred to be a multichamber container. The number of chambers in one container is not particularly limited, and may be determined depending on, for example, the number of components to be separately placed, and specifically, for example, 2 to 10 chambers, or 2 to 5 chambers. Specifically, for example, the container includes a first chamber for accommodating the composition and a second chamber for accommodating the other component. In the container, the first chamber and the second chamber may each be configured as an independent configuration, that is, configured as an independent container, or may be configured as an integrated configuration, that is, configured as a single container. When the first chamber and the second chamber are configured as a single container, the container is preferred to include a separating portion capable of separating the first chamber and the second chamber. When the container includes the separating portion, the first chamber and the second chamber are placed via the separating portion, for example. When the composition and the other components are mixed and administered to an administration subject, the separating portion is preferred to be configured so as to allow communication between the first chamber and the second chamber.

The container having the first chamber and the second chamber may be, for example, a medical multichamber container. The multichamber container may be, for example, a plastic double bag in which a plurality of chambers are formed by providing a separating barrier in a plastic bag (e.g., JP-A-2016-190646, and JP-A-2016-131577), a solution kit in which a container accomodating the other component and a container accomodating a solution (corresponds to the composition) are integrated so as to be communicatable with each other (e.g., WO 96/25136), a double-chamber prefilled syringe (e.g., JP-A-2012-245086), or the like.

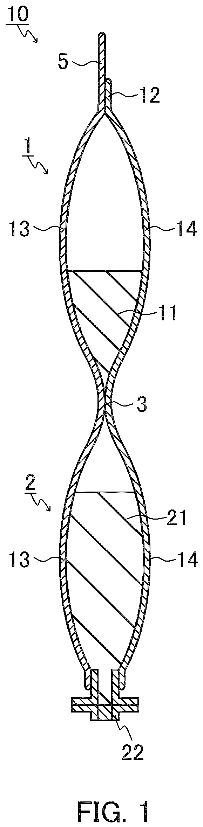

An example of the multichamber container accomodating the composition and the other component in the composition kit of the present invention will be described with reference to FIG. 1. FIG. 1 is a cross-sectional view showing an example of the composition kit of the present invention. As shown in FIG. 1, the composition kit includes a container 10, a composition 11, and an other component 21. The container 10 includes a first chamber 1 accomodating the composition 11, a second chamber 2 accomodating the other component 21, and a separating portion 3 that separates the first chamber 1 and the second chamber 2 and allows communication between the first chamber 1 and the second chamber 2. The container 10 further includes a hanging portion 5 capable of being used to suspend the container 10.

As shown in FIG. 1, the container 10 is formed of a sheet 13, a sheet 14, and a discharging portion (discharging port) 22. As shown in FIG. 1, the sheet 13 and the sheet 14 are welded to the sheet 13 at an upper end portion of the sheet 14 to form an upper end portion 12 of the first chamber 1, and connect to the discharging portion 22 at the lower end side of the sheet 13 and the sheet 14. Further, the sheet 13 and the sheet 14 are welded to each other at a central portion thereof to form a separating portion 3. The welding of the separating portion 3 is peelable. When the pressure is applied to the first chamber 1, the welding of the sheet 13 and the sheet 14 in the separating portion 3 is released, and the first chamber 1 and the second chamber 2 become communicatable with each other. In the container 10, the first chamber 1 is a space between the upper end portion 12 and the separating portion 3 in the sheet 13 and the sheet 14. In the container 10, the second chamber 2 is a space between the separating portion 3 and the discharging portion 22 in the sheet 13 and the sheet 14.

A plastic sheet may be used as the sheet 13 and the sheet 14. The plastic sheet is preferred to be composed of a plurality of layers, and includes, for example, an inner layer, an outer layer, and an intermediate layer. As the inner layer and the outer layer, for example, a thermoplastic resin such as a thermoplastic olefinic resin, a thermoplastic propylene resin, or a thermoplastic polyethylene resin can be used. Use of such a thermoplastic resin allows laminating the sheet 13 and the sheet 14 so as to face each other, and with the process of heat-sealing, outer peripheral portions of the first chamber 1 and the second chamber 2, the upper end portion 12 and the separating portion 3 can be easily formed, and thereby the container 10 can be produced. The intermediate layer is preferred to be, for example, a highly flexible resin, and specifically, for example, may be a thermoplastic olefinic resin composition.

The capacity and the shape of the first chamber 1 and the second chamber 2 are not particularly limited, and can be appropriately determined depending on, for example, the dosage of the composition and the other component.

The kit of the present invention can inhibit peritoneal deterioration of an administration subject. Therefore, the kit of the present invention can be suitably used, for example, as a kit for inhibiting peritoneal deterioration caused by peritoneal dialysis, preventing or inhibiting peritoneal inflammation and/or peritoneal fibrosis resulting from peritoneal deterioration, and encapsulating peritoneal sclerosis resulting from peritoneal deterioration.

### <Peritoneal dialysate>

In another aspect, the present invention provides a peritoneal dialysate that inhibits peritoneal deterioration. The peritoneal dialysate of the present invention is characterized by including the peritoneal deterioration inhibiting composition of the present invention. The peritoneal dialysate of the present invention can inhibit the peritoneal deterioration caused by peritoneal dialysis. The peritoneal dialysate of the present invention is characterized by including the composition of the present invention, i.e., CO as a gas component, and other configurations and conditions are not particularly limited. The peritoneal dialysate of the present invention can inhibit peritoneal deterioration caused in peritoneal dialysis. The composition of the present invention includes CO, and thus can be directly administered into a body of an administration subject, for example, peritoneally, intraperitoneally, or the like.

The peritoneal dialysate of the present invention includes an other component to be included in a peritoneal dialysate in addition to the composition of the present invention. That is, the peritoneal dialysate of the present invention includes the composition of the present invention, i.e., CO as a gas component, in addition to a component included as a general peritoneal dialysate. Examples of the other component include an osmotic pressure adjusting substance; ions of metals such as calcium, sodium, magnesium, and chromium; an alkalizing agent, and an organic acid.

Examples of an osmotic pressure adjusting substance include a sugar such as glucose and icodextrin, an amino acid, and a protein.

Examples of an alkalizing agent include lactate ion and bicarbonate ion.

Examples of an organic acid include propionic acid, malic acid, fumaric acid, succinic acid, oxalacetic acid, N-acetyl glycine, N-acetyl-L-cysteine, glutaric acid, glucuronic acid, ascorbic acid, citric acid, isocitric acid, gluconic acid, N-acetyl-L-aspartic acid, N-acetyl-L-glutamic acid, N-acetyl-L-methionine, N-acetyl-L-proline, N-acetyl-L-valine, N-acetyl-L-glutamine, N-acetyl-L-arginine, N-acetyl-L-histidine, N-acetyl-L-leucine, N-acetyl-L-tryptophane, and salts of these.

The concentration of the other component can be appropriately determined depending on the type of the other component.

The pH of the peritoneal dialysate of the present invention is, for example, about pH 5.0 to 7.5, and preferably about pH 6.5 to7.5.

The osmotic pressure of the peritoneal dialysate of the present invention is, for example, about 300 to 500 mOsm/kg, and preferably about 330 to 450 mOsm/kg.

The injection volume of the peritoneal dialysate of the present invention is, for example, 1.5 to 2 L per session when the subject is a human. The storage time of the peritoneal dialysate of the present invention is, for example, 4 to 8 hours per session. The peritoneal dialysate of the present invention is, for example, drained after the storage. In the peritoneal dialysate of the present invention, for example, counting a series of the above operations as one time, consecutive operations are continuously carried out 3 to 5 times per day.

The peritoneal dialysate of the present invention can be prepared, for example, by mixing the composition of the present invention with the other component such as the osmotic pressure adjusting substance. Therefore, it is preferrable that the other component is prepared as a concentrated liquid, and the concentration of the other component is adjusted so that the desired concentration can be achieved after the mixing with the composition of the present invention. The composition of the present invention and the other component are preferred to be sterilized in advance.

The peritoneal dialysate of the present invention is preferred to be encapsulated in a supple plastic bag, a glass container, or the like.

### <Peritoneal dialysate kit>

In another aspect, the present invention provides a peritoneal dialysate kit that inhibits peritoneal deterioration. The peritoneal dialysate kit of the present invention is characterized by including the composition of the present invention and a peritoneal dialysate. The peritoneal dialysate of the present invention can inhibit peritoneal deterioration caused in peritoneal dialysis. The peritoneal dialysate of the present invention is characterized by including the composition of the present invention, i.e., CO as a gas component, and other configurations and conditions are not particularly limited. The peritoneal dialysate of the present invention can inhibit peritoneal deterioration. The composition of the present invention includes CO and thus can be directly administered into a body of an administration subject, for example, peritoneally, intraperitoneally, or the like.

In the peritoneal dialysate kit of the present invention, the peritoneal dialysate is mixed with the composition of the present invention to prepare a mixed solution at the time of use, and the mixed solution is used. Therefore, the peritoneal dialysate is preferred to be, for example, a concentrated liquid of a normal peritoneal dialysate. The concentration of each component (other component) in the concentrated liquid of the peritoneal dialysate can be calibrated to the concentration which becomes a desired concentration after the mixing with the composition of the present invention.

In the peritoneal dialysate kit of the present invention, the composition and the other component are preferred to be accomodated in a container. The container can, for example, incorporate the description of the container in the composition kit described above.

### <Pharmaceutical composition>

In another aspect, the present invention provides a composition capable of treating a disease resulting from peritoneal dialysis or peritoneal deterioration. The pharmaceutical composition for treating a disease resulting from peritoneal dialysis or peritoneal deterioration of the present invention (hereinafter also referred to as the "pharmaceutical composition") includes the peritoneal deterioration inhibiting composition of the present invention. The pharmaceutical composition of the present invention is characterized by including the composition of the present invention, and other configurations and conditions are not particularly limited. The pharmaceutical composition of the present invention can inhibit peritoneal deterioration of an administration subject. Therefore, the pharmaceutical composition of the present invention can be suitably used, for example, as a pharmaceutical composition for inhibiting peritoneal deterioration caused by peritoneal dialysis, preventing or inhibiting peritoneal inflammation and/or peritoneal fibrosis caused in the process of the peritoneal deterioration, and encapsulating peritoneal sclerosis resulting from the peritoneal deterioration. The pharmaceutical composition of the present invention allows for the adjustment of the dosage of CO by adjusting the dosage of the composition.

The pharmaceutical composition of the present invention may include, for example, the pharmaceutically acceptable additive or the pharmaceutically acceptable carrier as described above.

### <Pharmaceutical kit>

In another aspect, the present invention provides a composition kit capable of treating a disease resulting from peritoneal dialysis or peritoneal deterioration. The pharmaceutical kit for treating a disease resulting from peritoneal dialysis or peritoneal deterioration of the present invention (hereinafter also referred to as the "pharmaceutical kit") includes the peritoneal deterioration inhibiting composition kit of the present invention, that is, the peritoneal deterioration inhibiting composition of the present invention and an other component. The pharmaceutical kit of the present invention is characterized by including the composition kit of the present invention and other configurations and conditions are not particularly limited. The pharmaceutical kit of the present invention can inhibit peritoneal deterioration of an administration subject. Therefore, the pharmaceutical kit of the present invention can be suitably used, for example, as a pharmaceutical composition for inhibiting peritoneal deterioration caused by peritoneal dialysis, preventing or inhibiting peritoneal inflammation and/or peritoneal fibrosis resulting from peritoneal deterioration, and encapsulating peritoneal sclerosis resulting from the peritoneal deterioration. The pharmaceutical kit of the present invention allows for adjusting the dosage of CO by adjusting the dosage of the composition.

### <Inhibition method>

In another aspect, the present invention provides a method of inhibiting peritoneal deterioration. The inhibition method of the present invention is a method for inhibiting peritoneal deterioration that uses the peritoneal deterioration inhibiting composition of the present invention, or the peritoneal deterioration inhibiting composition kit of the present invention. The inhibition method of the present invention is characterized by using the peritoneal deterioration inhibiting composition of the present invention or the peritoneal deterioration inhibiting composition kit of the present invention, and other processes and conditions are not particularly limited. The inhibition method of the present invention can inhibit peritoneal deterioration.

The inhibition method of the present invention includes, for example, administering the composition or the composition kit to a subject. The administration is preferably performed intraperitoneally.

In the inhibition method of the present invention, the subject may incorporate the above description of the administration subject, and specifically, for example, may be a subject who is scheduled to undergo peritoneal dialysis, a subject who is undergoing peritoneal dialysis, or a subject who has undergone peritoneal dialysis.

In the inhibition method of the present invention, the composition or the composition kit may be used *in vitro* or *in vivo.*

In the inhibition method of the present invention, the peritoneal deterioration may refer to, for example, peritoneal fibrosis, decrease in the peritoneal function, or the like.

### <Dialysis method>

In another aspect, the present invention provides a dialysis method in which peritoneal deterioration is inhibited. The dialysis method of the present invention is a peritoneal dialysis method which uses the peritoneal dialysate of the present invention or the peritoneal dialysate kit of the present invention. The dialysis method of the present invention is characterized by using the peritoneal dialysate of the present invention or the peritoneal dialysate kit of the present invention, and other processes and conditions are not particularly limited. The dialysis method of the present invention can inhibit peritoneal deterioration in peritoneal dialysis.

The dialysis method of the present invention includes, for example, injecting the peritoneal dialysate or a mixed solution of the peritoneal dialysate kit intraperitoneally to a subject, storing the peritoneal dialysate or the mixed solution, and draining the peritoneal dialysate or the mixed solution. The dialysis settings such as an injection amount and a storage time in each process can incorporate the above description.

### <Treatment method >

In another aspect, the present invention provides a method for treating a disease resulting from peritoneal dialysis or peritoneal deterioration. The treatment method of the present invention (hereinafter also referred to as the "treatment method") is a method for treating a disease resulting from peritoneal dialysis or peritoneal deterioration which uses the pharmaceutical composition of the present invention or the pharmaceutical composition kit of the present invention. The treatment method of the present invention is characterized by using the pharmaceutical composition of the present invention or the pharmaceutical composition kit of the present invention, and other processes and conditions are not particularly limited. The treatment method of the present invention can, for example, inhibit peritoneal deterioration of an administration subject in peritoneal dialysis. Therefore, the treatment method of the present invention can, for example, inhibit peritoneal deterioration caused by peritoneal dialysis, prevent or inhibit a disease such as peritoneal inflammation and/or peritoneal fibrosis caused by peritoneal deterioration, and encapsulating peritoneal sclerosis caused by peritoneal deterioration.

The treatment method of the present invention includes, for example, administering the peritoneal deterioration inhibiting composition of the present invention to a patient.

The treatment method of the present invention may use the pharmaceutical composition as the composition. The treatment method of the present invention can use the composition kit or the pharmaceutical kit (together hereinafter also referred to as the "kit") as the composition.

When the treatment method of the present invention uses the kit, the composition and the other component may be administered simultaneously or separately. When simultaneously administering the composition and the other component, the treatment method of the present invention preferably includes mixing the composition and the other component in the kit prior to the administering. In this case, the obtained mixture is to be administered to the patient.

The administration conditions in the administering can incorporate the above description.

In the treatment method of the present invention, the patient is preferably a patient diagnosed as having a disease resulting from peritoneal deterioration, or a patient suspected of having such disease. The patient may also be a patient having peritoneal fibrosis, a patient suspected of having peritoneal fibrosis, a patient having peritoneal inflammation, or a patient suspected of having peritoneal inflammation. Peritoneal deterioration can be evaluated, for example, by conducting a biopsy of the peritoneum of the patient.

### <Use>

In another aspect, the present invention is a composition for use in the inhibition of peritoneal deterioration, peritoneal dialysis, or treatment of a disease resulting from peritoneal dialysis or peritoneal deterioration, wherein the composition includes carbon monoxide. The present invention is also a composition kit for use in the inhibition of peritoneal deterioration, peritoneal dialysis, or treatment of a disease resulting from peritoneal dialysis or peritoneal deterioration, wherein the composition kit includes a composition and an other component, the composition and the other component are placed separately from each other, and the composition includes carbon monoxide.

### Examples

### [Example 1]

The composition of the present invention is produced to verify that the composition can reduce peritoneal deterioration.

### (1) Production of composition

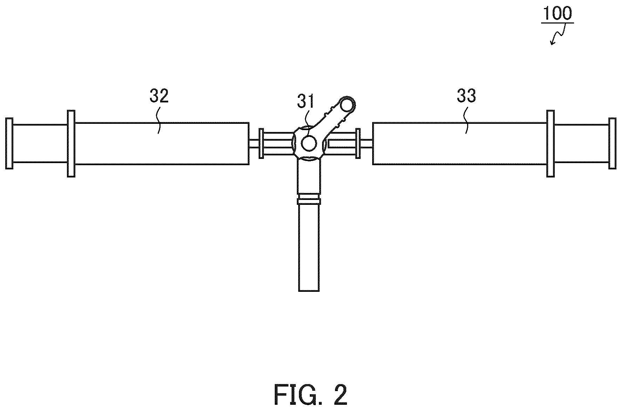

The composition of the present invention was produced using a type of micro bubble production equipment 100 shown in FIG. 2. As shown in FIG. 2, the production equipment 100 includes a syringe 32 and a syringe 33 disposed at two sides of a three-way stopcock 31. In the production equipment 100, the syringe 32 and the syringe 33 communicate with each other via a three-way stopcock 31. First, the syringe 32 was released from the three-way stopcock 31, and 20 mL of a physiological saline solution was introduced into the syringe 32. Then, the syringe 32 was reconnected to the three-way stopcock 31, and the gas in the three-way stopcock 31 was removed. After the removal, the syringe 33 was released from the three-way stopcock 31, and 20 mL of medical carbon monoxide (manufactured by TAIYO NIPPON SANSO CORPORATION or Japan Fine Products Corporation, CO concentration: 99.95% or more (G1)) was introduced into the syringe 33. Thereafter, the syringe 33 was reconnected to the three-way stopcock 31. After the reconnection, the plunger of each of the syringe 32 and the syringe 33 was continuously moved in piston motion in the outer cylinder for 10 minutes to produce micro bubbles including CO as a gas component, thereby producing the composition of the present invention (composition of Example 1).

The obtained composition was allowed to stand for about an hour, and then subjected to a measurement of its physical properties using NANOSIGHT^{™} NS300 (manufactured by Malvern Instruments) under default parameters. The measurement was performed at 25°C. As a result, the average diameter and density of the micro bubbles in the composition were as follows.

### (Composition of Example 1 (CO-UFB))

Average diameter: 136.3 ± 1.4 nm, density: 1.54× 10⁹ ± 1.87× 10⁷ bubbles/mL

### (2) Preparation of peritoneal deterioration model mice

A peritoneal deterioration model mouse was prepared by intraperitoneally administering a chlorhexidine gluconate solution (CG solution) to a mouse (strain: C57BL/6JJmsSlc, purchased from Japan SLC, Inc.). The CG solution was prepared as follows. First, 8.5 mL of a physiological saline solution was added with 1.5 mL of 99.5% ethanol (manufactured by FUJIFILM Wako Pure Chemical Corporation, Cat No:057-00456) and 50 µl of 20% CG (manufactured by FUJIFILM Wako Pure Chemical Corporation, Cat No:034-10871) and mixed thoroughly. The obtained mixed solution was sterilized with filtration through a 0.2 µm filter to prepare a 0.1% CG solution. Next, 300 µl of a 0.1% CG solution was intraperitoneally administered to the mouse. Two hours after the administration of the CG solution, 1.0 mL of CO-UFB was administered intraperitoneally. The administration was performed every other day, and a total of seven administrations were performed (CG+CO-UFB, example group). For the control group (Control), the administration was performed in the same manner as above except for using an equal amount of a physiological saline solution instead of the 0.1% CG solution, and a physiological saline solution instead of the CO-UFB. For the reference example group (CG), the administration was performed in the same manner as above except that a physiological saline solution was used instead of the CO-UFB.

### (3) Measurement of peritoneal function

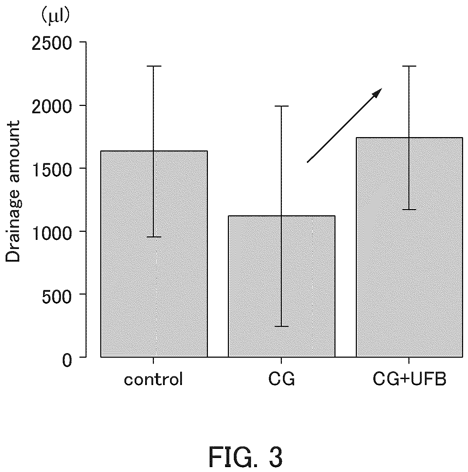

Sixteen days after the start of the administration, peritoneal function was measured to examine the occurrence of peritoneal deterioration. Specifically, the mouse of each group 16 days after the start of the administration was subjected to a peritoneal equilibration test (PET). The mouse of each group was subjected to intraperitoneal administration of 2 mL of 4.25% DIANEAL (registered trademark), and two hours after the administration, subjected to drainage and blood collection to evaluate the amount of removed water and the amount of drainage. These results are shown in Table 1 and FIG. 3.

**[Table 1]**

| | Control (n=13) | CG (n=16) | CG+CO-UFB (n=21) |
|---|---|---|---|
| Death rate (%) | 0% | 30.4% | 12.5% |
| Drainage amount (mL) | 1.634 mL | 1.120 mL | 1.741 mL |
| Water removal amount (mL) | -0.366 mL | -0.880 mL | -0.259 mL |

Table 1 shows death rates, drainage amounts, and water removal amount. FIG. 3 is a graph showing the drainage amounts. In FIG. 3, the horizontal axis indicates the type of samples, and the vertical axis indicates the drainage amount. As shown in Table 1, in the reference example group (CG) the death rate increased to 30.4% by induction of peritoneal deterioration. In contrast, in the example group (CG+CO-UFB) the death rate decreased to 12.5%. As shown in Table 1 and FIG. 3, the reference example group showed a decreasing tendency in the drainage amount as compared with the control group (control). In contrast, in the example group (CG+CO-UFB) the drainage amount was not significantly different from that in the control group.

From the above it was found that the administration of CO-UFB as the composition of the present invention can reduce decrease in water removal function caused by peritoneal deterioration.

### (4) Measurement of peritoneal thickening

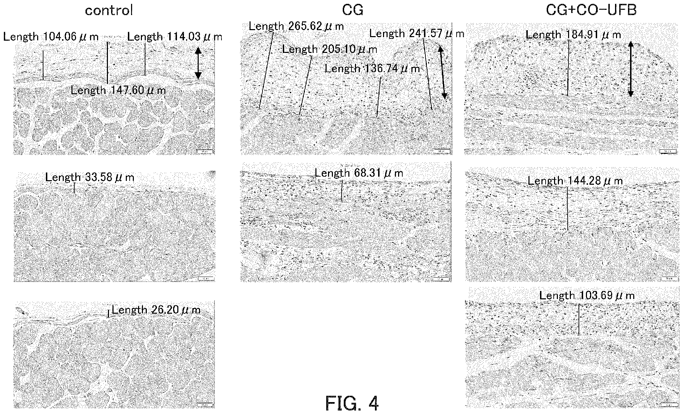

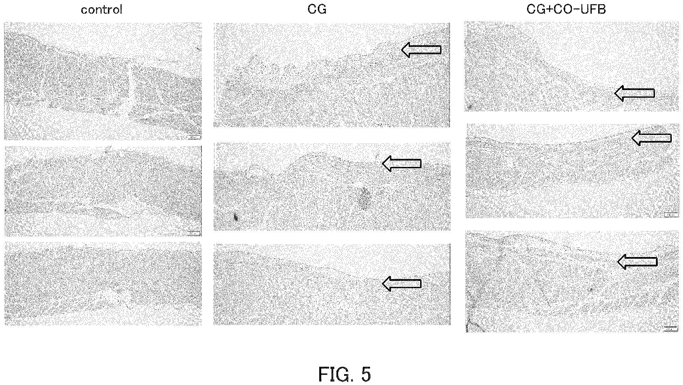

Sixteen days after the start of the administration of the CG solution in Example 1 (1), peritoneal thickening and infiltration of inflammatory cells into the peritoneum were examined as an index of peritoneal deterioration. Specifically, 16 days after the administration of the CG solution, the parietal peritoneum and the diaphragm of the mouse of each group were collected and fixed using 10% neutral buffered formalin. After the fixation, the parietal peritoneum and the diaphragm were paraffin-embedded, and paraffin-embedded sections having a thickness of 3 µm were prepared. The obtained paraffin-embedded sections were subjected to HE staining by an ordinary method. After the staining, the thickness between the basement membrane and the peritoneal surface (peritoneal surface layer) in each of the sections was measured using an optical microscope (OLYMPUS BX50, manufactured by Olympus Corporation) and a camera (OLYMPUS DP22, manufactured by Olympus Corporation). In addition, the occurrence of the infiltration of the inflammatory cells was examined in each section. These results are shown in FIG. 4 and FIG. 5.

FIG. 4 shows photographs of the thickening of the peritoneum stained using tissue staining. In FIG. 4, arrows indicate the region between the basement membrane and the peritoneal surface. FIG. 2 shows that the reference example group (CG) had an average peritoneal thickness of 166.6 µm, whereas the control group (control) had an average peritoneal thickness of 54.4 µm, which verified that the administration of the CG solution caused the thickening of the peritoneum. In contrast, the example group (CG+CO-UFB) had an average peritoneal thickness of 109.9 µm. These results showed that the administration of CO-UFB as the composition of the present invention can reduce peritoneal thickening that accompanies peritoneal deterioration.

FIG. 5 shows photographs of inflammatory cells stained using tissue staining. As shown in FIG. 5, no infiltration of the inflammatory cells into the peritoneal surface layer was observed in the control group (control). On the other hand, in the reference example group (CG) the infiltration of the inflammatory cells into the peritoneal surface layer was observed in the part indicated by the arrow, as compared with the control group. In contrast, in the example group (CG+CO-UFB), a decrease in the number of the inflammatory cells infiltrating into the peritoneal surface layer was observed in the part indicated by the arrow, as compared with the reference example group.

From the above it was found that the administration of CO-UFB as the composition of the present invention reduces the thickening of the peritoneum caused by peritoneal deterioration and inhibits the infiltration of the inflammatory cells.

### (5) Measurement of inflammatory response in peritoneal deterioration

Sixteen days after the start of the administration of the CG solution in Example 1 (1), the infiltration of macrophages, which are inflammatory cells, and the formation of blood vessels and lymphatic vessels related to the water removal ability were examined as an index of peritoneal deterioration. First, paraffin-embedded sections were prepared in the same manner as in Example 1 (4).

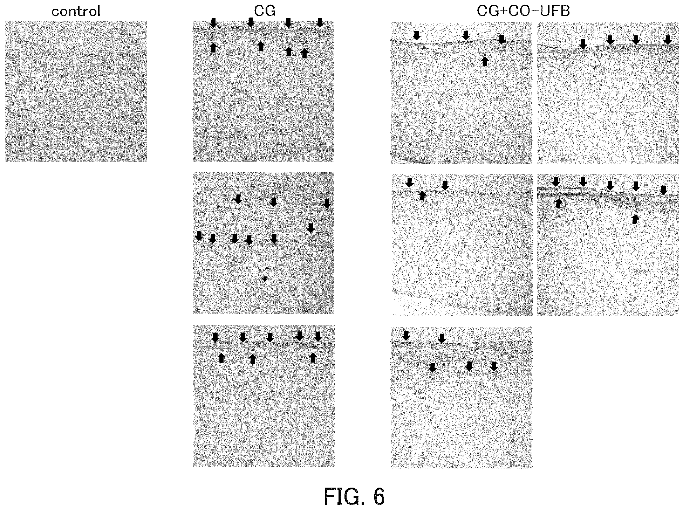

Next, the paraffin-embedded sections were reacted with a primary antibody, and then with a secondary antibody and an HRP label (HRP Rb, manufactured by Cell Signaling Technology, Inc., reagent code: 8114S, lot No: 21). As the primary antibody for staining macrophages, a CD68 antibody (Rt×Mo CD68, manufactured by Bio-Rad Laboratories, Inc., reagent code: MCA1957, lot No:1807-14) was used. As the primary antibody for staining vascular endothelial cells, a CD31 antibody (Rt×Mo CD31, manufactured by Merck, reagent code: CBL1337, lot No: 3123230) was used. As the primary antibody for staining lymphatic vessels, a Lyve-1 antibody (Rb×Mo/Rt Lyve-1, manufactured by Acris, reagent code: DP3513P, lot No: 1410R24) was used. After the reaction, staining was performed using DAB (3,3'-Diaminobenzidine) as a chromogenic substrate. After the staining, counterstaining was performed using hematoxylin. Then an optical microscope was used to tabulate the area in which CD68-positive macrophages are accumulated, the area filled with CD31-positive vascular endothelial cells, and the area filled with Lyve-1 positive lymphatic endothelial cells, and the proportion of each of the areas in one visual field was calculated. These results are shown in FIG. 6 to FIG. 11.

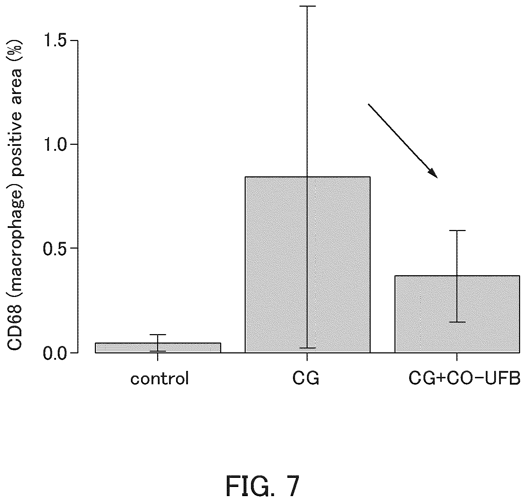

FIG. 6 shows photographs of the results of CD68 (macrophage marker) staining by immunohistochemical staining, and FIG. 7 is a graph showing macrophage positive areas (%). As shown in FIG. 6 and FIG. 7, no infiltration of macrophages was observed in the control group (control). On the other hand, in the reference example group (CG) the macrophage marker-positive area increased, as compared with the control group. In contrast, in the example group (CG+CO-UFB) the macrophage marker-positive area decreased as indicated by the arrow, as compared with the reference example group.

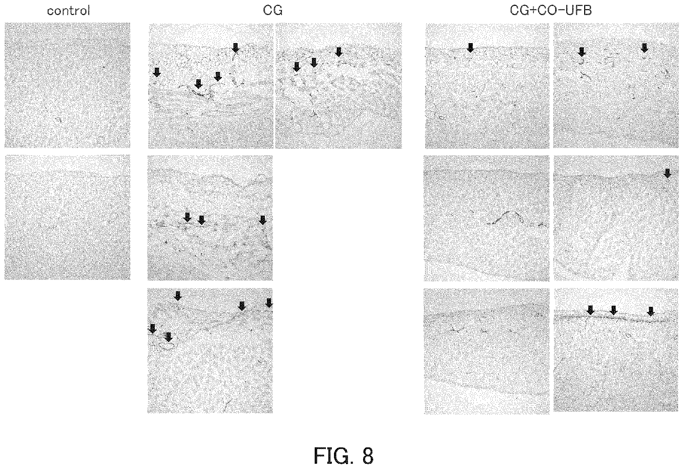

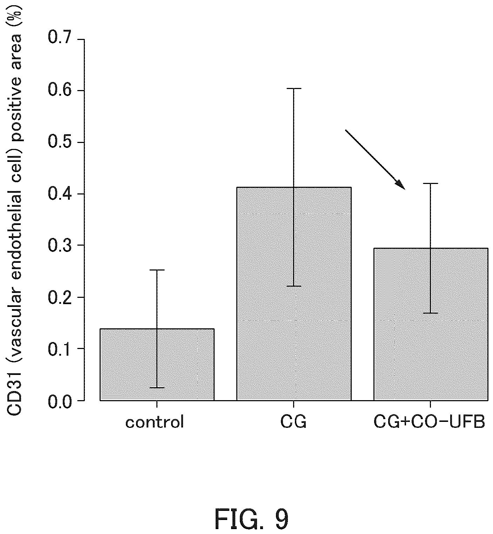

FIG. 8 shows photographs of the results of CD31 (vascular endothelial cell marker) staining by immunohistochemical staining, and FIG. 9 is a graph showing vascular endothelial cell-positive areas (%). As shown in FIG. 8 and FIG. 9, in the reference example group (CG) the vascular endothelial cell marker-positive area increased as indicated by the arrow, as compared with the control group (control). In contrast, the example group (CG+CO-UFB) showed a decreasing tendency in the vascular endothelial cell marker-positive area as indicated by the arrow, as compared with the reference example group.

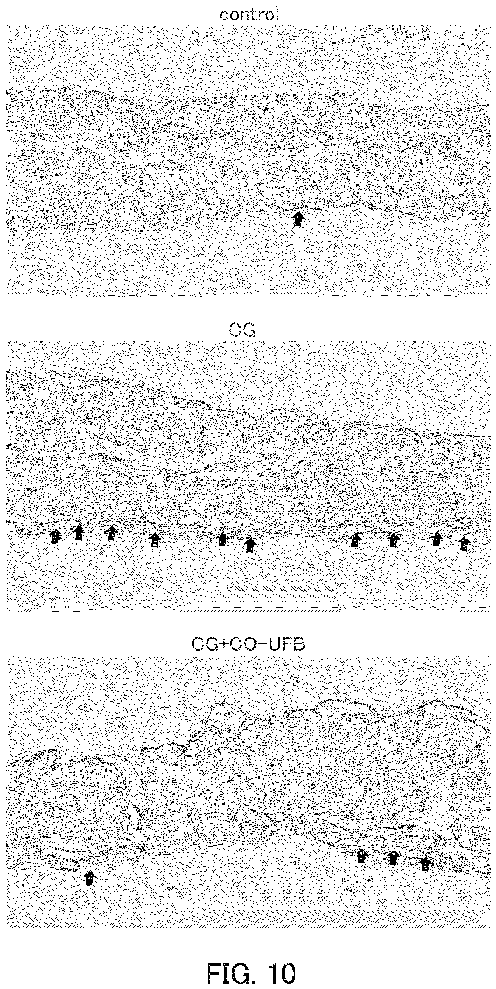

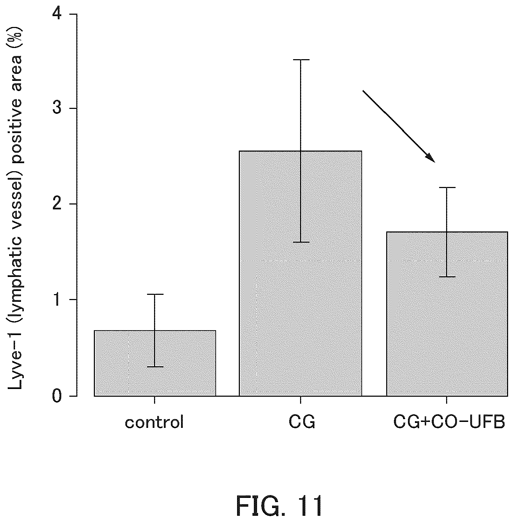

FIG. 10 shows photographs of the results of Lyve-1 (lymphatic vessel marker) staining by immunohistochemical staining, and FIG. 11 is a graph showing the results of lymphatic vessel-positive areas (%). In FIG. 10, the void portions are regions where lymphangiogenesis has occurred. As shown in FIG. 10 and FIG. 11, the reference example group (CG) showed an increasing tendency in the lymphatic marker-positive area as indicated by the arrow, as compared with the control group (Control). In contrast, the example group (CG+CO-UFB) showed a decreasing tendency in the lymphatic marker-positive area as indicated by the arrow, as compared with the reference example group.

These results showed that the administration of CO-UFB as the composition of the present invention inhibits the infiltration of macrophages of inflammatory cells and inhibits angiogenesis and lymphangiogenesis in the peritoneal deterioration model. As described above, peritoneal function such as water removal ability decreases in the peritoneal deterioration model. However, with the administration of CO-UFB, the decrease in peritoneal function is inhibited. This was presumed to be because CO-UFB inhibits inflammatory responses such as macrophage infiltration and the decrease in the water removal function due to angiogenesis and lymphangiogenesis, both of which occur in peritoneal deterioration.

### (6) Measurement of gene expression in peritoneal deterioration

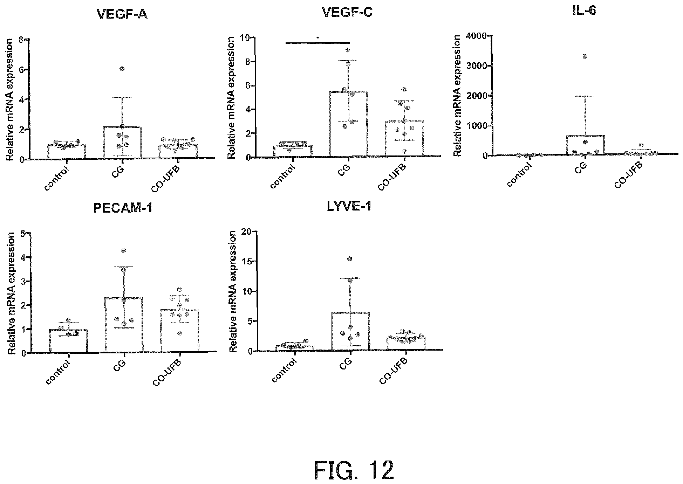

Sixteen days after the start of the administration of the CG solution in Example 1 (1), the gene expression related to angiogenesis, lymphangiogenesis, and inflammation was examined. Specifically, the parietal peritoneum and the diaphragm of the mouse of each group were collected, and Total RNA was extracted from each membrane by a conventional method. The obtained RNA, a reverse transcriptase (High Capacity cDNA Reverse Transcription Kit, manufactured by Applied Biosystems) and a PCR system (Thermal Cycler Dice, manufactured by Takara Bio Inc.) were used to synthesize cDNA. Then the cDNA, the primer set described below, a RT-PCR reagent (RTB GREEN^{™}, PREMIX^{™}, TAQ^{™} II, manufactured by Takara Bio Inc.) and a real-time PCR system (QuantStudio3, manufactured by Applied Biosystems) were used to measure the gene expression levels of VEGF-A (vascular endothelial growth factor), VEGF-C (lymphangiogenesis factor), PECAM-1 (platelet endothelial cell adhesion molecule), LYVE-1 (lymphatic endothelial cell marker), and IL-6 (inflammatory cytokine). GAPDH was used as the internal standard gene, and the relative expression levels to the expression level of the internal standard gene were calculated as the expression levels of each gene. These results are shown in FIG. 12.
· Primer set for VEGF-A
   Forward primer (SEQ ID NO: 1)
      5'-caggctgctgtaacgatgaa-3'
   Reverse primer (SEQ ID NO: 2)
      5'-gctttggtgaggtttgatcc-3'
·Primer set for VEGF-C
   Forward primer (SEQ ID NO: 3)
      5'-cagacaagttcattcaattattagacg-3'
   Reverse primer (SEQ ID NO: 4)
      5'-catgtcttgttagctgcctga-3'
·Primer set for PECAM-1
   Forward primer (SEQ ID NO: 5)
      5'-cggtgttcagcgagatcc-3'
   Reverse primer (SEQ ID NO: 6)
      5'-actcgacaggatggaaatcac-3'
·Primer set for LYVE-1
   Forward primer (SEQ ID NO: 7)
      5'-gaagcagctgggtttggag-3'
   Reverse primer (SEQ ID NO: 8)
      5'-cgtagcaaacagccagcac-3'
·Primer set for IL-6
   Forward primer (SEQ ID NO: 9)
      5'-gctaccaaactggatataatcagga-3'
   Reverse primer (SEQ ID NO: 10)
      5'-ccaggtagctatggtactccagaa-3'
·Primer set for the internal standard gene (GAPDH)
   Forward primer (SEQ ID NO: 11)
      5'-tgtgtccgtcgtggatctga-3'
   Reverse primer (SEQ ID NO: 12)
      5'-ttgctgttgaagtcgcaggag-3'

FIG. 12 is a graph showing the expression level of each gene. In FIG. 12, the horizontal axis indicates the type of samples, and the vertical axis indicates the expression level of each gene. As shown in FIG. 12, in the reference example group (CG) the expression levels of the vascular endothelial growth factor, the lymphangiogenesis factor, the platelet endothelial cell adhesion molecule, the lymphatic endothelial cell marker, and the inflammatory cytokine were increased, as compared with the control group (control). In contrast, in the example group (CG+CO-UFB) the expression levels of the vascular endothelial growth factor, the lymphangiogenesis factor, the platelet endothelial cell adhesion molecule, the lymphatic endothelial cell marker, and the inflammatory cytokine were decreased, as compared with the reference example Group.

These results show that the administration of CO-UFB as the composition of the present invention inhibits the expressions of vascular endothelial growth factors, lymphangiogenesis factors, platelet endothelial cell adhesion molecules, lymphatic endothelial cell markers, and inflammatory cytokines, which are to be enhanced in the peritoneal deterioration model. As described above, peritoneal function such as water removal ability is decreased in the peritoneal deterioration model. However, with the administration of CO-UFB, decrease in peritoneal function is inhibited. This was presumed to be because CO-UFB inhibits the enhancement of the gene expression associated with inflammatory responses such as macrophage infiltration, as well as decrease in water removal function due to angiogenesis and lymphangiogenesis, which occur in peritoneal deterioration.

### [Example 2]

The composition of the present invention was produced to verify that the composition can reduce peritoneal deterioration.

### (1) Production of composition

A composition including CO as micro bubbles was prepared in the same manner as in Example 1 (1) (composition of Example 2 (1)). The composition of Example 2 (1) was subjected to the measurements of the average diameter of the micro bubbles and the density of the micro bubbles in the composition in the same manner as in Example 1 (1) and showed the following results.

### (Composition of Example 2 (1) (CO-UFB))

Average diameter: 155.2 nm, density: 1.03×10⁸ bubbles/mL

A composition of a physiological saline solution in which CO is dissolved (composition of Example 2(2)) was prepared in the following manner. Specifically, the physiological saline solution and the medical carbon monoxide were encapsulated in a syringe at the volume ratio of 1:1 to dissolve the CO in the physiological saline solution by shaking and mixing for 30 minutes to prepare the composition of Example 2 (2). The result of the measurement of the concentration of carbon monoxide in the composition of Example 2 (2) was as follows.

### (Composition of Example 2 (2) (CO-Dissolve))

Concentration: 839 µM

### (2) Preparation of peritoneal deterioration model mice

Peritoneal deterioration model mice were prepared in the same manner as in Example 1 (2), except that the composition of Example 2 (1) and the composition of Example 2 (2) were administered instead of CO-UFB.

### (3) Measurement of peritoneal function

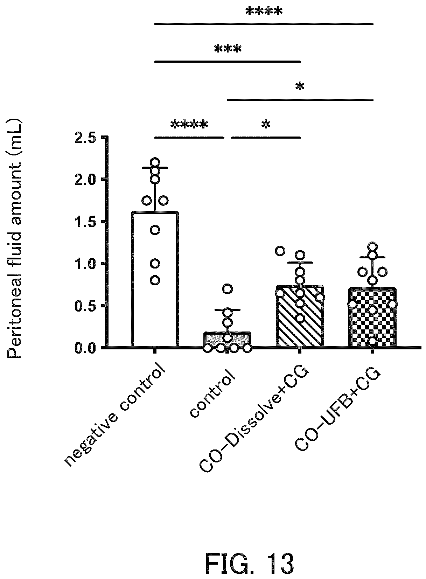

Next, peritoneal function was measured two days after the day of seventh administration (9 days after the start of CG solution administration). Peritoneal function was measured in the same manner as in Example 1 (3), and the amount of fluid in the peritoneal cavity (peritoneal fluid amount) of each mouse was calculated. The measurement for the negative control was performed in the same manner except that the mouse was untreated. The measurement for the control was performed in the same manner, except that a physiological saline solution was used instead of the composition of Example 2 (1) and the composition of Example 2 (2). These results are shown in FIG. 13. Note that in FIG. 13, * indicates p < 0.05, ** indicates p < 0.01, *** indicates p < 0.001, and **** indicates p < 0.0001.

FIG. 13 is a graph showing the peritoneal fluid amount. In FIG. 13 the horizontal axis indicates the type of samples, and the vertical axis indicates the peritoneal fluid amount (mL). As shown in FIG. 13 the control (control) had largely reduced water removal amount, as compared with the negative control (negative control). In contrast, in the administration groups of the composition of Example 2 (1) (CO-UFB+CG) and the composition of Example 2 (2) (CO-Dissolve+CG), decrease in water removal amount was significantly reduced as compared with the control, and showed that decrease in water removal function due to peritoneal deterioration can be reduced.

### (4) Measurement of peritoneal thickening

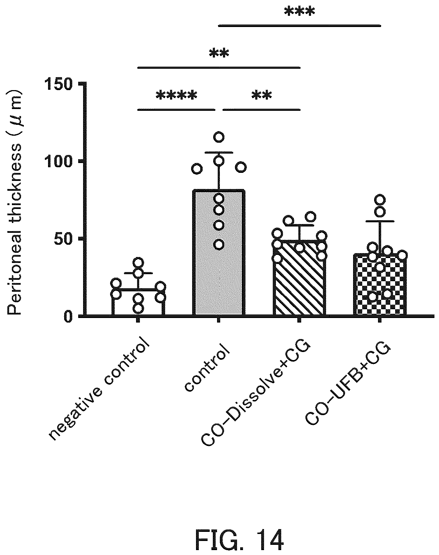

Next, the thickness of the peritoneal surface was measured two days after the day of seventh administration (nine days after the start of CG solution administration), as the index of peritoneal deterioration. The measurement of the thickness of the peritoneal surface was performed in the same manner as in Example 1 (4). The results are shown in FIG. 14.

FIG. 14 is a graph showing the thickness of the peritoneal surface (peritoneal thickness). In FIG. 14 the horizontal axis indicates the type of samples, and the vertical axis indicates the thickness of the peritoneal surface. As shown in FIG. 14, the control (control) had largely increased peritoneal thickness as compared with the negative control (negative control). In contrast, in the administration groups of the composition of Example 2 (1) (CO-UFB+CG) and the composition of Example 2 (2) (CO-Dissolve+CG), increase in peritoneal thickness was significantly reduced as compared with the control, and showed that the peritoneal thickening caused in peritoneal deterioration can be reduced. Further, increase in peritoneal thickness of the administration group of the composition of Example 2 (1) tended to be further reduced, as compared with the administration group of the composition of Example 2 (2).

### [Example 3]

The composition of the present invention was produced to verify that the composition can prevent peritoneal deterioration.

### (1) Production of composition

A composition including CO as micro bubbles and a composition of a physiological saline solution in which CO is dissolved were prepared in the same manner respectively as the composition of Example 1 (1) and the composition of Example 2 (1) (the composition of Example 3 (1) and the composition of Example 3 (2), respectively). The composition of Example 3 (1) was subjected to the measurements of the average diameter of the micro bubble and the density of the micro bubbles in the composition in the same manner as in Example 1 (1) and showed the following results. The composition of Example 3 (2) was subjected to the measurement of the density of carbon monoxide in the composition and showed the following result.
(Composition of Example 3 (1) (CO-UFB))
   Average diameter: 155.2 nm, density: 1.03×10⁸ bubbles/mL
(Composition of Example 3 (2) (CO-Dissolve))
   CO concentration: 755 µM

### (2) Prevention of peritoneal deterioration

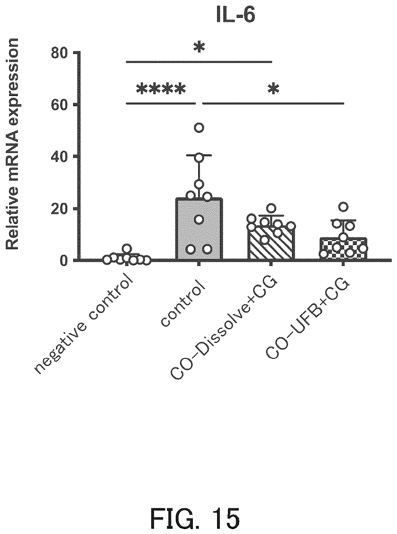

Whether or not the composition of the present invention can prevent peritoneal deterioration was examined by administering the composition of each of the Examples prior to administration of CG. Specifically, first, 1.0 mL of the composition of Example 3 (1) or the composition of Example 3 (2) was intraperitoneally administered to the mouse. One hour after the administration, 300 µL of 0.1% CG solution was intraperitoneally administered to the mouse. Six hours after the administration of the CG solution, the parietal peritoneum and the diaphragm of each mouse were collected. The relative expression level of IL-6 genes was calculated in the same manner as in Example 1 (6), except that the parietal peritoneum and the diaphragm were used. The examination for the negative control was performed in the same manner as above except that the mouse was untreated. The examination for the control was performed in the same manner as above except that a physiological saline solution was used instead of the composition of Example 3 (1) and the composition of Example 3 (2). These results are shown in FIG. 15.

FIG. 15 is a graph showing expression levels of IL-6 genes. In FIG. 15 the horizontal axis indicates the type of the samples, and the vertical axis indicates the expression level of IL-6 genes. As shown in FIG. 15, the control (control) showed a largely increased expression level of IL-6 genes as compared with the negative control (negative control). In contrast, the administration groups of the composition of Example 3 (1) (CO-UFB+CG) and the composition of Example 3 (2) (CO-Dissolve+CG) showed significantly decreased expression levels of IL-6 genes as compared with the control and showed that the inflammation that occurs before peritoneal deterioration can be reduced. Further, increase in the expression level of IL-6 genes of the administration group of the composition of Example 3 (1) tended to be further reduced, as compared with the administration group of the composition of Example 3 (2).

### [Example 4]

A dilution series of the composition of the present invention was produced to examine the effect of the CO concentration on the reduction of peritoneal deterioration.

### (1) Production of composition

A composition including CO as micro bubbles was prepared in the same manner as the composition of Example 1 (1). Thereafter, as compositions of Example 4 (1), an undiluted solution, a fourfold diluted solution, a tenfold diluted solution, and a fiftyfold diluted solution were prepared (CO-UFB, CO-UFB 1/4, CO-UFB 1/10, CO-UFB 1/50, respectively). Further, a composition of a physiological saline solution in which CO is dissolved was prepared in the same manner as the composition of Example 2 (2). Thereafter, as the compositions of Example 4 (2), an undiluted solution, a fourfold diluted solution, a tenfold diluted solution, and a fiftyfold diluted solution were prepared (CO-dis, CO-dis 1/4, CO-dis 1/10, CO-dis 1/50, respectively).

### (2) Preparation of peritoneal deterioration model mice

Peritoneal deterioration model mice were prepared in the same manner as in Example 1 (2), except that the compositions of Example 4 (1) or the compositions of Example 4 (2) were administered instead of CO-UFB.

### (3) Measurement of peritoneal function

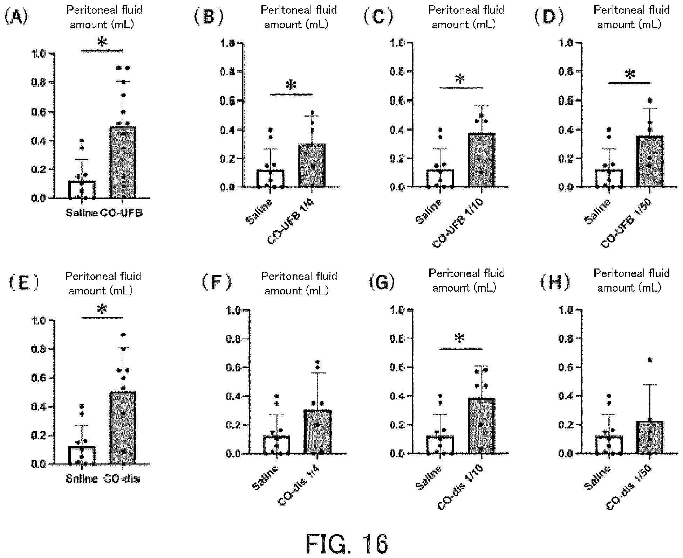

Peritoneal Equilibration Test (PET) was performed in the same manner as in Example 1 (3), except that peritoneal function was measured 14 days after the start of administration to calculate the amount of fluid in the peritoneal cavity (peritoneal fluid amount) of each mouse. The measurement for the control (Saline) was performed in the same manner as above except that a physiological saline solution was used instead of the compositions of Example 4 (1) or the compositions of Example 4 (2). These results are shown in FIGs. 16A to 16H. Note that * in FIGs. 16A to 16H indicates p < 0.05.

FIGs. 16A to 16H are graphs showing the peritoneal fluid amounts. In FIGs. 16A to 16H, the horizontal axis indicates the type of the samples, and the vertical axis indicates the peritoneal fluid amount (mL). The peritoneal fluid amount representing the permeability of the peritoneum was higher in the groups administered the compositions of the Examples than in the control (Saline). In particular, the groups administered the compositions of the Examples (CO-UFB, CO-UFB 1/4, CO-UFB 1/10, and CO-UFB 1/50) had significantly a higher peritoneal fluid amount than the control (Saline). With respect to the groups administered the compositions of Example 4 (2), FIGs. 16E and 16G (CO-dis and CO-dis 1/10) showed a significantly higher peritoneal fluid amount than the control (Saline). The average peritoneal fluid amounts in FIGs. 16F and 16H (CO-dis 1/4 and CO-dis 1/50) were higher than that of the control (Saline). These results showed that the composition of the present invention can help to counteract decrease in water removal function due to peritoneal deterioration, even at low concentrations of CO.

While the present invention has been described above with reference to illustrative embodiments, the present invention is by no means limited thereto. Various changes and variations that may become apparent to those skilled in the art may be made in the configuration and specifics of the present invention without departing from the scope of the present invention.

### <Supplementary Notes>

The whole or part of the example embodiments disclosed above can be described as, but not limited to, the following supplementary notes.

### <Peritoneal deterioration inhibiting composition>

### (Supplementary Note 1)

A composition for use in the inhibition of peritoneal deterioration, including carbon monoxide.

### (Supplementary Note 2)

The composition according to Supplementary Note 1, further including micro bubbles, wherein the micro bubble includes carbon monoxide as a gas component.

### (Supplementary Note 3)

The composition according to Supplementary Note 2, wherein
density of the micro bubbles is 5×10⁵ to 5×10¹² bubbles/mL.

### (Supplementary Note 4)

The composition according to Supplementary Note 2 or 3, wherein
proportion of the carbon monoxide in the gas component is 80% or more.

### (Supplementary Note 5)

The composition according to any one of Supplementary Notes 2 to 4, wherein
the gas component in the micro bubble is surrounded by an aqueous solvent.

### (Supplementary Note 6)

The composition according to any one of Supplementary Notes 2 to 5, further including a medium, wherein
the medium is at least one of liquid or solid.

### (Supplementary Note 7)

The composition according to Supplementary Note 1, further including a medium, wherein
the carbon monoxide is dissolved in the medium.

### (Supplementary Note 8)

The composition according to Supplementary Note 7, wherein
content of the carbon monoxide dissolved in the medium is 0.01 µmol/L to 5 mmol/L.

### (Supplementary Note 9)

The composition according to any one of Supplementary Notes 1 to 8, wherein
the peritoneal deterioration is peritoneal fibrosis.

### (Supplementary Note 10)

The composition according to any one of Supplementary Notes 1 to 9, wherein
the peritoneal deterioration is a decrease in peritoneal function.

### (Supplementary Note 11)

The composition according to any one of Supplementary Notes 1 to 10 for use in intraperitoneal administration.

### <Peritoneal deterioration inhibiting composition kit>

### (Supplementary Note 12)

A composition kit for use in the inhibition of peritoneal deterioration, including:
a composition, and
an other component, wherein
the composition and the other component are placed separately from each other, and
the composition is the composition according to any one of Supplementary Notes 1 to 11.

### (Supplementary Note 13)

The composition kit according to Supplementary Note 12, further including a container, wherein the container includes a plurality of chambers and a separating portion for separating the respective chambers,
the plurality of chambers include at least a first chamber and a second chamber,
the composition is accommodated in the first chamber,
the other component is accommodated in the second chamber, and
the separating portion separates the first chamber and the second chamber and allows
communication between the first chamber and the second chamber.

### (Supplementary Note 14)

The composition kit according to Supplementary Note 12 or 13, wherein
the other component includes an osmotic pressure adjusting substance.

### < Peritoneal dialysate>

### (Supplementary Note 15)

A peritoneal dialysate, including the composition according to any one of Supplementary Notes 1 to 11.

### < Peritoneal dialysate kit>

### (Supplementary Note 16)

A peritoneal dialysate kit, including:
a composition, and
a peritoneal dialysate, wherein
the composition and the peritoneal dialysate are placed separately from each other, and
the composition is the composition according to any one of Supplementary Notes 1 to 11.

### (Supplementary Note 17)

The peritoneal dialysate kit according to Supplementary Note 16, further including a container, wherein
the container includes a plurality of chambers and a separating portion for separating the respective chambers,
the plurality of chambers include at least a first chamber and a second chamber,
the composition is accommodated in the first chamber,
the peritoneal dialysate is accommodated in the second chamber, and
the separating portion separates the first chamber and the second chamber and allows
communication between the first chamber and the second chamber.

### <Composition for treating disease resulting from peritoneal dialysis or peritoneal deterioration>

### (Supplementary Note 18)

A pharmaceutical composition for use in treatment of a disease resulting from peritoneal dialysis or peritoneal deterioration, including carbon monoxide.

### (Supplementary Note 19)

The pharmaceutical composition according to Supplementary Note 18, further including micro bubbles, wherein
the micro bubble includes carbon monoxide as a gas component.

### (Supplementary Note 20)

The pharmaceutical composition according to Supplementary Note 19, wherein
density of the micro bubbles is 5×10⁵ to 5×10¹² bubbles/mL.

### (Supplementary Note 21)

The pharmaceutical composition according to Supplementary Note 19 or 20, wherein
the proportion of the carbon monoxide in the gas component is 80% or more.

### (Supplementary Note 22)

The pharmaceutical composition according to any one of Supplementary Notes 19 to 21, wherein
the gas component in the micro bubble is surrounded by an aqueous solvent.

### (Supplementary Note 23)

The pharmaceutical composition according to any one of Supplementary Notes 19 to 22, further including a medium, wherein
the medium is at least one of a liquid or a solid.

### (Supplementary Note 24)

The pharmaceutical composition according to Supplementary Note 18, further including a medium, wherein
the carbon monoxide is dissolved in the medium.

### (Supplementary Note 25)

The pharmaceutical composition according to any one of Supplementary Notes 19 to 24, wherein
the disease resulting from peritoneal dialysis is at least one of peritoneal fibrosis, peritonitis,
and/or encapsulating peritoneal sclerosis.

### (Supplementary Note 26)

The pharmaceutical composition according to any one of Supplementary Notes 19 to 25 for use in intraperitoneal administration.

### <Composition kit for treating disease resulting from peritoneal dialysis or peritoneal deterioration>

### (Supplementary Note 27)

A pharmaceutical composition kit for use in treatment of a disease resulting from peritoneal dialysis or peritoneal deterioration, including:
a composition, and
an other component, wherein
the composition and the other component are placed separately from each other, and
the composition is the composition according to any one of Supplementary Notes 1 to 11.

### (Supplementary Note 28)

The pharmaceutical composition kit according to Supplementary Note 27, further including a container, wherein
the container includes a plurality of chambers and a separating portion for separating the respective chambers,
the plurality of chambers include at least a first chamber and a second chamber,
the composition is accommodated in the first chamber,
the other component is accommodated in the second chamber, and
the separating portion separates the first chamber and the second chamber and allows
communication between the first chamber and the second chamber.

### (Supplementary Note 29)

The pharmaceutical composition kit according to Supplementary Note 27 or 28, wherein
the other component includes an osmotic pressure adjusting substance.

### <Method for inhibiting peritoneal deterioration>

### (Supplementary Note 30)

A method for inhibiting peritoneal deterioration using the composition according to any one of Supplementary Notes 1 to 11, or the composition kit according to any one of Supplementary Notes 12 to 14.

### (Supplementary Note 31)

The method according to Supplementary Note 30, including
administering the composition or the composition kit to a subject.

### (Supplementary Note 32)

The method according to Supplementary Note 31, wherein
the administration is performed intraperitoneally.

### (Supplementary Note 33)

The method according to Supplementary Note 31 or 32, wherein
the subject is a subject who is scheduled to undergo peritoneal dialysis, a subject who is undergoing peritoneal dialysis, or a subject who has undergone peritoneal dialysis.

### (Supplementary Note 34)

The method according to any one of Supplementary Notes 30 to 33, wherein
the composition or the composition kit is used *in vitro* or *in vivo.*

### (Supplementary Note 35)

The method according to any one of Supplementary Notes 30 to 34, wherein
the peritoneal deterioration is peritoneal fibrosis.

### (Supplementary Note 36)

The method according to any one of Supplementary Notes 30 to 35, wherein
the peritoneal deterioration is a decrease in peritoneal function.

### <Peritoneal dialysis method>

### (Supplementary Note 37)

A peritoneal dialysis method using the peritoneal dialysate according to Supplementary Note 15, or the peritoneal dialysate kit according to Supplementary Note 16 or 17.

### (Supplementary Note 38)

The dialysis method according to Supplementary Note 37, including:
injecting the peritoneal dialysate or a mixed solution of the peritoneal dialysate kit intraperitoneally in a subject;
storing the peritoneal dialysate or the mixed solution, and
draining the peritoneal dialysate or the mixed solution.

### <Method for treating disease resulting from peritoneal dialysis or peritoneal deterioration>

### (Supplementary Note 39)

A method for treating a disease resulting from peritoneal dialysis or peritoneal deterioration using the pharmaceutical composition according to any one of Supplementary Notes 18 to 26, or the pharmaceutical composition kit according to any one of Supplementary Notes 27 to 29.

### (Supplementary Note 40)

The method according to Supplementary Note 39, including
administering the pharmaceutical composition or the pharmaceutical composition kit to a subject.

### (Supplementary Note 41)

The method according to Supplementary Note 40, wherein
the administration is performed intraperitoneally.

### (Supplementary Note 42)

The method according to Supplementary Note 40 or 41, wherein
the subject is a subject who is scheduled to undergo peritoneal dialysis, a subject who is undergoing peritoneal dialysis, or a subject who has undergone peritoneal dialysis.

### (Supplementary Note 43)

The method according to any one of Supplementary Notes 39 to 42, wherein
the composition or the composition kit is used *in vitro* or *in vivo.*

### (Supplementary Note 44)

The method according to any one of Supplementary Notes 39 to 43, wherein
the peritoneal deterioration is peritoneal fibrosis.

### (Supplementary Note 45)

The method according to any one of Supplementary Notes 39 to 44, wherein
the peritoneal deterioration is a decrease in peritoneal function.

### (Supplementary Note 46)

The method according to any one of Supplementary Notes 39 to 45, wherein
the disease resulting from peritoneal dialysis is at least one of peritoneal fibrosis, peritonitis,
and/or encapsulating peritoneal sclerosis.

### (Supplementary Note 47)

The method according to any one of Supplementary Notes 39 to 46, wherein
the treatment is prevention or inhibition.

### <Use>

### (Supplementary Note 48)

A composition for use in the inhibition of peritoneal deterioration, including the composition according to any one of Supplementary Notes 1 to 11.

### (Supplementary Note 49)

A composition kit for use in the inhibition of peritoneal deterioration, including:
a composition, and
an other component, wherein
the composition and the other component are placed separately from each other, and
the composition is the composition according to any one of Supplementary Notes 1 to 11.

### (Supplementary Note 50)

A composition for use in peritoneal dialysis, including the composition according to any one of Supplementary Notes 1 to 11.

### (Supplementary Note 51)

A composition kit for use in peritoneal dialysis, including:
a composition, and
an other component, wherein
the composition and the other component are placed separately from each other, and
the composition is the composition according to any one of Supplementary Notes 1 to 11.

### (Supplementary Note 52)

A composition for use in treatment of a disease resulting from peritoneal dialysis or peritoneal deterioration, wherein
the composition includes the composition according to any one of Supplementary Notes 1 to 11.

### (Supplementary Note 53)

A composition kit for use in treatment of a disease resulting from peritoneal dialysis or peritoneal deterioration, including:
a composition, and
an other component, wherein
the composition and the other component are placed separately from each other, and
the composition is the composition according to any one of Supplementary Notes 1 to 11.

### Industrial Applicability

As described above, the present invention can inhibit peritoneal deterioration. Therefore, the present invention can, for example, inhibit the peritoneal deterioration caused by peritoneal dialysis, and prevent or inhibit encapsulating peritoneal sclerosis due to the peritoneal deterioration. Accordingly, the present invention can be suitably used, for example, in the treatment of diseases caused by peritoneal deterioration, and is extremely useful in the medical field, the pharmaceutical field, and the like.

This application claims priority from Japanese Patent Application No. 2022-100790 filed on June 23, 2022. The entire subject matter of the Japanese Patent Application is incorporated herein by reference.

### Reference Signs List

- 1: first chamber
- 10: container
- 11: composition
- 12: upper end portion
- 13, 14: sheet
- 2: second chamber
- 21: other component
- 22: discharging portion
- 3: separating portion
- 5: hanging portion

Sequence List

## Claims

1. A composition for use in inhibition of peritoneal deterioration, comprising carbon monoxide.

2. The composition according to Claim 1, further comprising micro bubbles, wherein
the micro bubble includes carbon monoxide as a gas component.

3. The composition according to Claim 2, wherein
density of the micro bubbles is 5×10⁵ to 5×10¹² bubbles/mL.

4. The composition according to Claim 2 or 3, wherein
proportion of the carbon monoxide in the gas component is 80% or more.

5. The composition according to Claim 2 or 3, wherein
the gas component in the micro bubble is surrounded by an aqueous solvent.

6. The composition according to Claim 2 or 3, further comprising a medium,
wherein the medium is at least one of liquid or solid.

7. The composition according to Claim 1, further comprising a medium, wherein
the carbon monoxide is dissolved in the medium.

8. The composition according to Claim 7, wherein
content of the carbon monoxide dissolved in the medium is 0.01 µmol/L to 5 mmol/L.

9. The composition according to any one of Claim 1, 2, 7, or 8, wherein
the peritoneal deterioration is peritoneal fibrosis.

10. The composition according to any one of Claim 1, 2, 7, or 8, wherein
the peritoneal deterioration is decrease in peritoneal function.

11. The composition according to any one of Claim 1, 2, 7, or 8 for use in intraperitoneal administration.

12. A composition kit for use in inhibition of peritoneal deterioration, comprising:
a composition, and
an other component, wherein
the composition and the other component are placed separately from each other, and
the composition is the composition according to any one of Claim 1, 2, 7, or 8.

13. The composition kit according to Claim 12, further comprising a container, wherein
the container includes a plurality of chambers and a separating portion for separating the respective chambers,
the plurality of chambers include at least a first chamber and a second chamber,
the composition is accommodated in the first chamber,
the other component is accommodated in the second chamber, and
the separating portion separates the first chamber and the second chamber and allows communication between the first chamber and the second chamber.

14. The composition kit according to Claim 12, wherein
the other component includes an osmotic pressure adjusting substance.

15. A peritoneal dialysate, comprising the composition according to any one of Claim 1, 2, 7, or 8.

16. A peritoneal dialysate kit, comprising:
a composition, and
a peritoneal dialysate, wherein
the composition and the peritoneal dialysate are placed separately from each other, and
the composition is the composition according to any one of Claim 1, 2, 7, or 8.

17. The peritoneal dialysate kit according to Claim 16, further comprising a container, wherein
the container includes a plurality of chambers and a separating portion for separating the respective chambers,
the plurality of chambers include at least a first chamber and a second chamber,
the composition is accommodated in the first chamber,
the peritoneal dialysate is accommodated in the second chamber, and
the separating portion separates the first chamber and the second chamber and allows communication between the first chamber and the second chamber.

18. A pharmaceutical composition for use in prevention or inhibition of a disease resulting from peritoneal dialysis, comprising carbon monoxide.

19. A pharmaceutical composition kit for use in prevention or inhibition of a disease resulting from peritoneal dialysis, comprising:
a composition, and
an other component, wherein
the composition and the other component are placed separately from each other, and
the composition is the composition according to any one of Claim 1, 2, 7, or 8.
